(19) Europäisches Patentamt
European Patent Office
Office européen des brevets

(11) Publication number: **0 009 302**
**B1**

(12) **EUROPEAN PATENT SPECIFICATION**

(45) Date of publication of patent specification: **26.10.83**

(51) Int. Cl.[3]: **C 07 D 498/04,**
A 61 K 31/42 //(C07D498/04, 263/00, 205/00)

(21) Application number: **79301285.7**

(22) Date of filing: **02.07.79**

(54) **Clavulanic acid derivatives and process for their preparation.**

(30) Priority: **09.09.78 GB 3627078**

(43) Date of publication of application:
**02.04.80 Bulletin 80/7**

(45) Publication of the grant of the patent:
**26.10.83 Bulletin 83/43**

(84) Designated Contracting States:
**AT BE CH DE FR GB IT LU NL SE**

(56) References cited:
**EP - A - 0 005 014**
**DE - A - 2 646 003**
**DE - A - 2 817 085**

(73) Proprietor: **BEECHAM GROUP PLC**
**Beecham House Great West Road**
**Brentford Middlesex (GB)**

(72) Inventor: **Stirling, Irene**
**80 Woodcrest Walk**
**Reigate, Surrey (GB)**
Inventor: **Clarke, Brian Peter**
**"Coneybury", Drive Spur, Forest Drive**
**Kingswood, Surrey (GB)**

(74) Representative: **Hesketh, Alan, Dr. et al,**
**European Patent Attorney Beecham Pharmaceuticals Great Burgh Yew Tree Bottom Road**
**Epsom, Surrey, KT18 5XQ (GB)**

**EP 0 009 302 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Clavulanic acid derivatives and process for their preparation

Our earlier cognate British Patent Application No. 16764/77 — 37072/77 — 50229/77 — 53866/77 (and also French Patent Application No. 78—11 851, West German Patent Application No. P.28 17 085.6, Japan Patent Application No. 48292/78 and U.S.A Patent Application No. 896 441) disclosed inter alia a process for the preparation of the compounds of the formula (I):

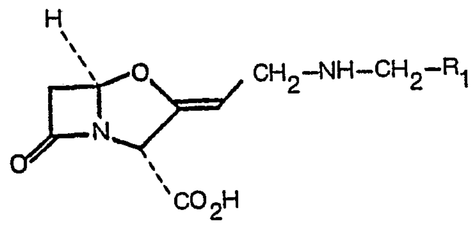

(I)

wherein $R_1$ is a hydrogen atom, an alkyl group of up to 5 carbon atoms, a cycloalkyl group of 5 or 6 carbon atoms, a hydroxyalkyl group of up to 5 carbon atoms or a moiety of the sub formula (a):

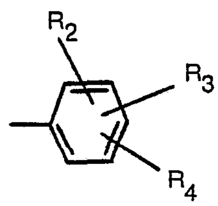

(a)

wherein $R_2$ is a hydrogen, fluorine, chlorine or bromine atom or an alkyl group of 1—3 carbon atoms, an alkoxyl group of 1—3 carbon atoms, an acyloxyl group of 1—3 atoms, a hydroxyl group, an alkoxycarbonyl group containing 1—3 carbon atoms in the alkoxy part, or a group $-N(R_5)CO.R_6$, $-N(R_5)SO_2R_6$ or $-CO-NR_5R_6$ where $R_5$ is a hydrogen atom or an alkyl group of 1—3 carbon atoms or a phenyl or benzyl group and $R_6$ is an alkyl group of 1—3 carbon atoms or a phenyl or benzyl group; $R_3$ is a hydrogen, fluorine or chlorine atom or an alkyl group of 1—3 carbon atoms, an alkoxyl group of 1—3 carbon atoms or an acyloxyl group of 1—3 carbon atoms; and $R_4$ is a hydrogen fluorine or chlorine atom or an alkyl group of 1—3 carbon atoms or an alkoxyl group of 1—3 carbon atoms. The disclosed process comprised inter alia the hydrogenation of a compound of the formula (II):

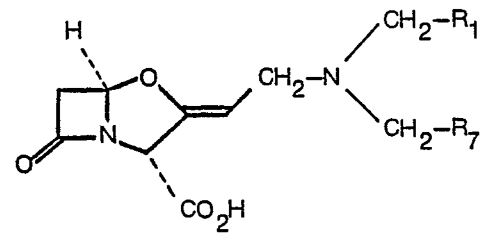

(II)

or a hydrogenolysable ester thereof wherein $R_1$ is as defined in relation to formula (I) and $R_7$ is a group of the sub-formula (a) as defined in relation to formula (I).

It has now been discovered that the compounds of the formula (I) may also be prepared by the catalytic removal of substituted allyl groups from compounds of analogous to those of formula (II) in which the $CH_2R_7$ moiety is replaced by a substituted allyl group. This process can improve yields of product and can be more convenient in that quicker reaction times and lower pressures of hydrogen may be employed.

The present invention provides a process for the preparation of a compound of the formula (I) as hereinbefore defined which process comprises the catalytic hydrogenation of a compound of the formula (III):

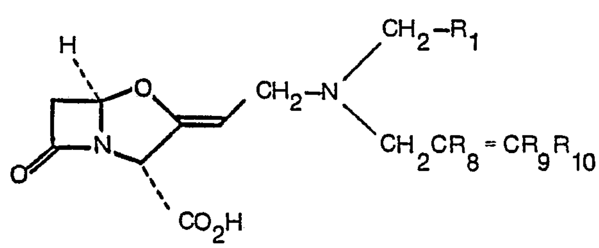

(III)

of a hydrogenolysable ester thereof wherein $R_1$ is as defined in relation to formula (I), or $R_1$ is $R_1^1$ is a moiety that on hydrogenation provides a group $R_1$ as defined in formula (I), $R_8$ is a lower alkyl group or a hydrogen atom, $R_9$ is a hydrogen atom or a lower alkyl group and $R_{10}$ is a hydrogen atom or a lower alkyl or phenyl group.

When used herein the term "hydrogenolysable ester" means an ester which on hydrogenation is cleaved to yield the parent carboxylic acid.

When used herein the term "lower alkyl" means an alkyl group of up to 4 carbon atoms. Thus examples of suitable alkyl groups are the methyl, ethyl, n-propyl, iso-propyl, n-butyl, sec-butyl and iso-butyl groups. Favoured lower alkyl groups include the methyl and ethyl groups. A preferred lower alkyl group is the methyl group.

Suitably $R_1$ is a hydrogen atom. Suitably $R_1$ is an alkyl group of up to 5 carbon atoms. Suitably $R_1$ is a hydroxyalkyl group of up to 5 carbon atoms. Suitably $R_1$ is a phenyl group optionally substituted by a fluorine, chlorine or bromine atom or an alkyl or alkoxyl group of up to 3 carbon atoms.

Apt groups $R_1$ include the methyl, ethyl, propyl, butyl, hydroxymethyl, hydroxyethyl, hydroxypropyl, phenyl, p-methoxyphenyl, p-methylphenyl and the like groups. Certain particularly apt groups $R_1$ include the methyl, ethyl.

Other suitable groups $R_1$ include those of the sub-formula (b):

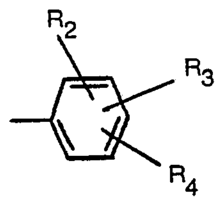

(b)

wherein $R_2$ is a hydrogen, fluorine, chlorine or bromine atom or an alkyl group of 1—3 carbon atoms, an alkoxyl group of 1—3 carbon atoms, an acyloxy group of 1—3 carbon atoms, a hydroxyl group or an alkoxycarbonyl group containing 1—3 carbon atoms in the alkoxy part; $R_3$ is a hydrogen, fluorine or chlorine atom or an alkyl group of 1—3 carbon atoms, an alkoxyl group of 1—3 carbon atoms or an acyloxyl group of 1—3 carbon atoms; and $R_4$ is a hydrogen, fluorine or chlorine atom or an alkyl group of 1—3 carbon atoms or an alkoxyl group of 1—3 carbon atoms.

More suitably $R_2$ is a hydrogen, fluorine or chlorine atom or a methoxyl, ethoxyl, hydroxyl, acetoxyl, propionyloxyl, methyl, ethyl, methoxycarbonyl or ethoxycarbonyl group.

More suitably $R_3$ is a hydrogen, fluorine or chlorine atom or a methoxyl, ethoxyl, acetoxyl, propionoxyl methyl or ethyl group.

More suitably $R_4$ is a hydrogen, fluorine or chlorine atom or a methoxyl, ethoxyl, acetoxyl, propionoxyl, methyl or ethyl group.

More suitably $R_2$ is a hydrogen, fluorine or chlorine atom or a methoxyl, hydroxyl or methyl group.

Most suitably $R_3$ is a hydrogen, fluorine or chlorine atom or a methoxyl or methyl group.

Most suitably $R_4$ is a hydrogen atom or a methyl or methoxyl group.

Preferably $R_2$ is a hydrogen, fluorine or chlorine atom or a methyl or methoxyl group.

Preferably $R_3$ is a hydrogen atom or methoxyl group.

Preferably $R_4$ is a hydrogen atom.

Certain particularly favoured values for $R_1$ include those of the sub-formula (c):

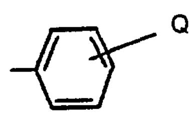

(c)

wherein Q is a hydrogen, fluorine or chlorine atom or a methyl, methoxyl, ethyl or ethoxyl group.

Suitably Q is a hydrogen, *p*-fluorine, *m*-fluorine, *p*-chlorine or *m*-chlorine atom or a *p*-methyl, *m*-methyl, *p*-methoxyl or *m*-methoxyl group.

Most suitably Q is a hydrogen, *p*-fluorine or *p*-chlorine atom or a *p*-methyl or *p*-methoxyl group.

Particularly preferably Q is a hydrogen atom. It has been found that the process of this invention is particularly advantageous when employed in the preparation of 9-benzylaminodeoxyclavulanic acid.

A further favoured group of values for $R_1$ is that of the sub-formula (d):

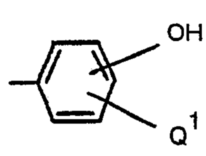

(d)

wherein $Q^1$ is a hydrogen, fluorine or chlorine atom or a methyl, ethyl, methoxyl, ethoxyl or hydroxyl group.

Suitably the OH substituent shown in formula (d) is para- to the carbon to which the —NH—$CH_2$— moiety is attached.

Suitably the OH substituent shown in formula (d) is meta- to the carbon atom to which the —NH—$CH_2$— moiety is attached.

Most suitably $Q^1$ is a hydrogen atom or a methyl or methoxyl group.

Yet another favoured group of values for $R_1$ is that of the sub-formula (e):

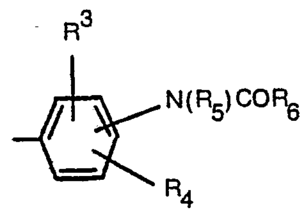

(e)

wherein $R_3$, $R_4$, $R_5$ and $R_6$ are as defined in relation to formula (I):

Suitably $R_5$ a hydrogen atom or an alkyl group of 1—3 carbon atoms such as a methyl group and most suitably $R_5$ is a hydrogen atom. More suitably $R_6$ is an alkyl group of 1—3 carbon atoms such as the methyl group.

Favoured values for $R_3$ and $R_4$ in relation to sub formula (e) are those as defined in relation to sub-formula (b). Preferably $R_3$ and $R_4$ are both hydrogen atoms.

Suitably in sub-formula (e) the —$N(R_5)COR_6$ moiety is attached para to the —NH—$CH_2$— moiety.

Suitably in sub-formula (e) the —$N(R_5)COR_6$ moiety is attached meta to the —NH—$CH_2$— moiety.

A particularly favoured group of values for $R_1$ is that of the sub-formula (f):

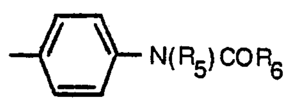

(f)

wherein $R_5$ and $R_6$ are as defined in relation to sub-formula (e). Particularly suitably $R_5$ is a hydrogen atom or an alkyl group of 1— 3 carbon atoms such as a methyl group. Preferably $R_5$ is a hydrogen atom. Particularly suitably $R_5$ is an alkyl group of 1—3 carbon atoms and preferably a methyl group.

Particularly apt groups $CH_2CR_8{=}CR_9R_{10}$ for use in the compounds of the formula (III) include the following $CH_2C(CH_3){=}CH_2$, $CH_2C(C_2H_5){=}CH_2$, $CH_2C(n.C_3H_7){=}CH_2$, $CH_2C(CH_3){=}CH.CH_3$, $CH_2C(CH_3){=}C(CH_3)_2$, $CH_2C(CH_3){=}CH.C_2H_5$ and $CH_2.C(CH_3){=}CH.C_6H_5$.

A favoured group $CH_2$ $CR_8{=}CR_9R_{10}$ is the $CH_2C(CH_3){=}CH_2$ group.

A further favoured group $CH_2CR_8{=}CR_9R_{10}$ is the $CH_2C(CH_3){=}CHPh$ group.

The compound of the formula (III) may be pre-formed although it is normally produced in-situ by the hydrogenation of a hydrogenolysable ester. It follows that it is a preferred form of the process of this invention to employ a hydrogenolysable ester of a compound of the formula (III).

Hydrogenolysable esters for use in this invention are generally benzyl or substituted benzyl esters: and also optionally substituted allyl esters. Suitable esters include those of the part-formula (g): $CO_2CHA_1A_2$ wherein $A_1$ is a hydrogen atom or a lower alkyl or optionally substituted phenyl group and $A_2$ is an optionally substituted phenyl group.

Favourably $A_1$ is a hydrogen atom.

The nature of the substituent employed in a substituted phenyl group is unimportant as long as it does not interfere with the hydrogenolytic cleavage. Thus suitable substituents include lower alkyl, lower alkoxyl, lower acyloxyl, lower acyl, nitro, cyano, carboxylic acid groups or salts or lower alkyl esters or amides thereof, nitro, halo or similar substituents. Apt substituents include methyl, methoxyl, nitro, chloro, bromo and the like. One, two or three such substituents may be employed (except not more than one nitro group should be present).

Favoured esters include the benzyl, nitrobenzyl, bromobenzyl, chlorobenzyl, methylbenzyl and methoxybenzyl esters. Particularly favoured esters include the benzyl, p-nitrobenzyl and p-methoxybenzyl esters. The preferred ester is the benzyl ester.

Further preferred hydrogenolysable ester groups include those groups $CH_2CR_8 = CHR_{10}$ that have been specified hereinbefore as being favoured for removal from a nitrogen atom by hydrogenolysis.

The hydrogenation reaction is normally carried out in the presence of a transition metal catalyst.

The catalyst we have preferred to use is palladium, for example in the form of palladium on carbon (charcoal), palladium on barium sulphate, palladium on calcium carbonate, palladium black or the like.

A favoured catalyst is palladium on carbon (sometimes referred to as palladium on charcoal); for example 5%, 10%, 20% or 30% palladium on carbon.

The higher palladium content catalyst can be particularly apt as smaller total weights of catalyst can be employed thereby avoiding possible problems associated with adsorption of product onto the carbon. Alternatively use of a catalyst containing a lower proportion of metal and a higher quantity of

carbon can be used specifically to adsorb the product. This aids in isolation as the product may be recovered therefrom thereafter by washing.

A low, medium or high pressure of hydrogen may be used in this reaction, for example from 1 to 6 atmospheres.

However it is one of the considerable advantages of the process of this invention that it proceeds smoothly and quickly even at atmospheric pressure. Thus a particularly favoured aspect of this invention comprises carrying out the hydrogenation at atmospheric pressure.

The reaction is normally carried out at a non-extreme temperature, for example from 0°C to 30°C and more usually from 12°C to 25°C. It is generally convenient to carry out the reaction at ambient temperature.

Suitable solvents for carrying out the hydrogenation include ethanol, n-propanol, isopropanol, tetrahydrofuran, dioxan, ethyl acetate or mixtures of such solvents or such solvents in the presence of water. A favoured solvent is ethanol.

The product may generally be isolated from the reaction mixture by filtering off the solids (the catalyst, which should be well washed to remove the product) and then evaporating the solvent, preferably under low pressure, to yield the initial product. Further purification may be effected by such conventional methods as chromatography over cellulose or other mild stationary phase eluting with a $C_{1-4}$ alkanol optionally in the presence of water and optionally in the presence of tetrahydrofuran. Evaporation of the combined active fraction (identified by aqueous potassium permanganate spray on tlc) then yields the desired compound in pure form. The desired product is normally obtained in crystalline form (unless it is an unsalted ester). Trituration under ethanol, isopropanol or the like $C_{1-4}$ alkanol or other conventional solvent such as a ketone, ether or ester solvent or other conventional solvent (for example of up to 6 carbon atoms and more suitably of up to 4 carbon atoms) may also be used to aid crystallisation. Recrystallisation from ethanol or the like may also be employed. The solvent used in such processes may advantageously be moist.

The compounds of the formula (III) as hereinbefore defined and their hydrogenolysable esters are useful intermediates and such form part of this invention.

The compounds of the formula (III) and their hydrogenolysable esters may be prepared by the methods set forth in British Patent Application No. 41887/75, U.S. Serial No. 731928, Belgian Patent No. 847044 and West German Offenlegungsschrift No. 26460037. In summary an apt form of this process comprises the reaction of an hydrogenolysable ester of an acyl derivative of clavulanic acid with an amine of the formula (IV):

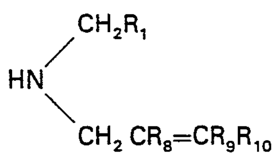

(IV)

wherein $R_1$, $R_8$, $R_9$ and $R_{10}$ are as defined in relation to formula (III) and thereafter cleaving the ester if desired.

A favoured acyl derivative is the dichloroacetate. A favoured ester is the benzyl ester.

The following Examples illustrate the invention.

## Example 1

Benzyl 9-N-(2′-methylallyl)benzylaminodeoxyclavulanate

Benzyl dichloroacetylclavulanate (5 g; 12.5 mM) in dimethylformamide (70 $cm^3$) at 0° was treated with *N*-(2-methylallyl)benzylamine (1.9 equivalents) dropwise with stirring. The mixture was stirred for 3 hours at 0° then poured into ethylacetate (250 $cm^3$) and washed with water (5 × 150 $cm^3$) and saturated brine (5 × 100 $cm^3$) dried (anhydrous magnesium sulphate) and evaporated in vacuo to yield an oil. This crude product was chromatographed on silica eluting with cyclohexane/ethylacetate; 1:1. Fractions (detected by aqueous potassium permanganate spray) were collected containing the title compound Rf ($SiO_2$/ethylacetate:cyclohexane; 1:1) = 0.84. Combined fractions were evaporated to yield an oil 2.8 g (36%). $\nu$ (film) 1805, 1750, 1695, 1495, 1455, 1308, 1230, 1175, 1120, 1015, 895, 745, 700 $cm^{-1}$. $\delta$ ($CDCl_3$) 1.70 (3H, s), 2.85 (2H, s), 2.76—3.10 (1H, d), 3.08 (2H, *J* 7 Hz), 3.40 (1H, dd, *J* 17 and 3 Hz) 3.41 (2H, s), 4.71 (1H, t, *J* 7 Hz), 4.75—4.95 (2H, broad m), 5.03 (1H, broad s) 5.15 (2H, s) 5.57 (1H, d, *J* 3 Hz), 7.25 and 7.30 (10H, 2 × s).

The intermediate *N*-(2-methylallyl)benzylamine was prepared as follows:

Benzaldehyde (7.42 g; 70 mM) in 150 $cm^3$ of a solvent mixture of ethylacetate/chloroform/ethanol was treated with 1 equivalent of 2-methylallyl amine and stirred for $\frac{1}{2}$ hour. The mixture was treated with a slight excess of sodium borohydride and stirred for $\frac{1}{2}$ hour. The solvent was removed by evaporation in vacuo and the residue was redissolved in ethylacetate (200 $cm^3$) then washed with water (3 × 100 $cm^3$). The free amine was extracted into aqueous hydrochloric acid, the aqueous phase was washed with ethyl acetate (2 × 100 $cm^3$). Fresh ethylacetate was added (150 $cm^3$) and stirred vigorously with sodium carbonate until alkaline. The ethylacetate phase containing the free

amine was washed with water (5 x 100 cm³) and saturated brine (3 x 100 cm³), dried (anhydrous magnesium sulphate) and evaporated to a mobile colourless liquid, yield = 8.1 g (72%) $\nu$ (film) 3330, 1650, 1495, 1450, 1115, 1030, 895, 735, 700 $cm^{-1}$. $\delta$ ($CDCl_3$) 1.40 (1H, s, exchanges with $D_2O$), 1.74 (3H, s) 3.15 (2H, s) 3.70 (2H, s) 4.76—4.95 (2H, m), 7.25 (5H, m). $C_{11}H_{15}N$ requires 161.1180; 161.1192 found.

## Example 2

9-N-Benzylaminodeoxyclavulanic acid

Benzyl 9-*N*-(2-methylallyl)benzylaminodeoxyclavulanate (0.5 g) was hydrogenolysed in neat ethanol (30 $cm^3$) in the presence of palladium on carbon (10%; 0.2 g; prehydrogenated for 20 minutes) for 25 minutes at $1.013 \times 10^5$ Pa (1 atmosphere). The catalyst was filtered off and washed with ethanol (10 $cm^3$), the catalyst was then washed with aqueous ethanol (50%; 150 $cm^3$), this aqueous ethanolic wash was collected separately and evaporated to yield a white crystalline solid. This solid was washed with cold (0°) ethanol and dried to yield 164 mg (49%) of the title compound. Rf ($SiO_2$/butanol-propan-2-ol-water; 7:7:6) = 0.45 (detection by aqueous potassium permanganate spray. $\nu$ (Nujol) 1815, 1690, 1612, 1575, 1305, 1187, 1125, 1082, 1065, 1045, 1035, 1018, 1005, 990, 950, 892, 750, 695 $cm^{-1}$.

## Example 3

Benzyl 9-N-(4′-acetamidobenzyl)-N-(2′-methylallyl) aminodeoxyclavulanate

Benzyl dichloroacetyl clavulanate (3.2 g; 8 mm), in dry dimethylformamide (30 $cm^3$) at 0°C was treated with 1.9 equivalents of *N*-(4-acetamidobenzyl)-N-(2′-methylallyl)amine and stirred for $2\frac{1}{4}$ hours at 0°C. The mixture was poured into ethyl acetate (250 $cm^3$) and washed with water (4 x 100 $cm^3$) and saturated brine (4 x 100 $cm^3$), dried (anhydrous magnesium sulphate) and evaporated to an oil. This oil was chromatographed on silica gel eluting with ethyl acetate. Fractions were collected containing the title compound, Rf ($SiO_2$/ethylacetate/cyclohexane 1:1) = 0.33 (detection by aqueous potassium permanganate spray). Combined fractions were evaporated to give a foam, yield = 1.92 g (49%), $\nu$ (film) 3320 (broad), 1805, 1750, 1695, 1670, 1605, 1535, 1515, 1455, 1415, 1370, 1310, 1265, 1230, 1175, 1120, 1040, 1020, 895, 830, 750, 700 $cm^{-1}$, $\delta$ ($CDCl_3$) 1.69 (3H, s), 2.12 (3H, s), 2.84 (2H, s), 2.75—3.07 (1H, m), 3.06 (2H, d, *J* 7 Hz), 3.36 (2H, s), 3.40 (1H, dd, *J* 17 and 3 Hz), 4.69 (1H, t, *J* 7 Hz), 4.74—4.98 (2H, broad m), 5.02 (1H, s), 5.15 (2H, s), 5.58 (1H, d, *J* 3Hz), 7.13—7.45 (10H, m).

## Example 4

9-N-(4′-Acetamidobenzyl)aminodeoxyclavulanic acid

Benzyl 9-*N*-(4′-acetamidobenzyl)-*N*-(2′-methylallyl) aminodeoxyclavulanate (1.14 g; 2.33 mm) in tetrahydrofuran — ethanol (1:1; 40 $cm^3$) was hydrogenolysed for 4 hours at $1.013 \times 10^5$ Pa (1 atmosphere) in the presence of 10% palladium on carbon (0.3 g). The catalyst was filtered and washed with aqueous ethanol (30 $cm^3$) and the filtrate evaporated to an oil. This oil was dissolved in tetrahydrofuran-ethanol (50%; 10 $cm^3$) and cooled (0°) slowly; crystals formed which were filtered off and washed with cold (0°) tetrahydrofuran-ethanol mixture (1:1) and dried to yield the title compound as white crystals, yield = 172 mg (21%) Rf ($SiO_2$/butanol-propan-2-ol-water; 7:7:6) = 0.45.

$\nu$ (NUJOL) (1810—1790), 1692, 1675, 1600 $cm^{-1}$. $\delta$ ($D_2O$) 2.12 (3H, s), 3.04 (1H, d, *J* 17 Hz), 3.53 (1H, dd, *J* 17 Hz and 3 Hz), 3.69 (2H, d, *J* 7 Hz), 4.12 (2H, s), 4.76 (1H, t, *J* 7 Hz), 4.94 (1H, s), 5.69 (1H, d, *J* 3 Hz), 7.40 (4H, s).

## Example 5

Benzyl 9-*N*-(2′-methyl-3′-phenylallyl)ethylaminodeoxyclavulanate

Benzyl dichloroacetylclavulanate (5 g; 12.5 mM) in dry dimethylformamide (70 $cm^3$) at −10° was treated with 1.9 equivalents of *N*-ethyl-*N*-(2-methyl-3-phenylallyl)amine and stirred for 30 minutes allowing the temperature to rise slowly to −2°. The mixture was poured into iced ethylacetate (250 $cm^3$) and washed with water (5 x 100 $cm^3$) and saturated brine (5 x 100 $cm^3$), dried (anhydrous magnesium sulphate) and evaporated to an oil. This oil was dissolved in toluene (15 $cm^3$) and chromatographed on silica eluting with ethyl acetate — cyclohexane; 1:2. Fractions were collected containing the title compound, Rf ($SiO_2$/ethyl acetate — cyclohexane; 1:1) = 0.5 (detection by aqueous potassium permanganate spray). Combined fractions were evaporated to yield an oil; 1.4 g (25%), $\nu$ (film) 1805, 1750, 1700, 1308, 1175, 1015, 745, 700 $cm^{-1}$. $\delta$ ($CDCl_3$) 1.00 (3H, t, *J* 7 Hz), 1.84 (3H, d, *J* 1 Hz), 2.42 (2H, q, *J* 7 Hz), 2.93 (1H, d, *J* 17 Hz), 2.96 (2H, s), 3.17 (2H, d, *J* 7 Hz), 3.39 (1H, dd, *J* 17 and 3 Hz), 4.73 (1H, t, *J* 7 Hz), 5.16 (1H, s), 7.25 and 7.32 (10H, 2 x broad s).

## Example 6

9-*N*-Ethylaminodeoxyclavulanic acid

Benzyl 9-*N*-(2′-methyl-3′-phenylallyl)ethylaminodeoxyclavulanate (0.71 g; 1.59 mM) in ethanol (40 $cm^3$) was hydrogenolysed at $1.013 \times 10^5$ Pa (1 atmosphere) in the presence of palladium on carbon (10%), 250 mg (which had been prehydrogenated for 15 minutes) for 1 hour. The catalyst was

filtered off and washed with aqueous ethanol (50 $cm^3$), the filtrate was evaporated and ethanol was added, the resulting crystalline solid was filtered off cold (0°) and washed with a little cold ethanol. Drying afforded the title compound as a white crystalline solid, yield = 78 mg. The filtrate was again evaporated, cold methanol was added and the resulting crystalline solid filtered off, washed with cold methanol and dried to yield a further 10 mg of the title compound; total yield = 88 mg (24%). Rf ($SiO_2$/butanol -isopropanol — water; 7:7:6) = 0.38 (detection by aqueous potassium permanganate spray). $\nu$ (Nujol) (3700—2000) very broad, 1808, 1695, 1620, 1585, 1302, 1190, 1120, 1045, 1020, 1008, 925, 895, 870, 802, 750 $cm^{-1}$. $\nu$ (KBr) (3700—3140), (3140—2890), (2890—2600), (2540—2100), 1790, 1690 (1675—1510), 1470, 1390, 1375, 1300, 1185, 1118, 1042, 1018, 920, 895, 803, 752 $cm^{-1}$.

$\delta$ ($D_2O$) 1.21 (3H, t, *J* 7 Hz), 3.02 (2H, q, *J* 7 Hz), 3.07 (1H, d, *J* 17 Hz), 3.56 (1H, dd, *J* 17 and 3 Hz), 3.68 (2H, d, *J* 7.5 Hz), 4.77 (1H, broad, t, *J* 7.5 Hz), 4.96 (1H, s), 5.74 (1H, d, *J* 3 Hz).

## Example 7

Benzyl 9-N-(2″-methylallyl)-N-(1′,2′,3′,6′-tetrahydrobenzyl)aminodeoxyclavulanate

Benzyl dichloroacetylclavulanate (6 g; 15 mM) in dry dimethylformamide (40 $cm^3$) at 0° was treated with *N*-(2″-methylallyl)-*N*-(1′,2′,3′,6′-tetrahydrobenzyl) amine (1.9 equivalents) and stirred at 0° for 1½ hours. The mixture was poured into ethyl acetate (200 $cm^3$) and washed with water (5 x 100 $cm^3$) and saturated brine (5 x 100 $cm^3$), dried (anhydrous magensium sulphate) and evaporated to yield an oil. This oil was chromatographed on silica gel eluting with ethyl acetate-cyclohexane; 1:1. Fractions were collected containing the title compound, Rf ($SiO_2$/ethylacetate-cyclohexane; 1:1) = 0.9 (detection by aqueous potassium permanganate spray). Combined fractions were evaporated to an oil, yield = 3.2 g (48%). $\nu$ (film) 1805, 1750, 1695, 1450, 1305, 1175, 1120, 1042, 1010, 895, 745, 700, 655 $cm^{-1}$.

$\delta$ ($CDCl_3$) 1.00—2.30 (7H, broad m), 1.67 (3H, s), 2.14 (2H, d, *J* 6 Hz), 2.80 (2H, s), 2.96 (1H, d, *J* 17 Hz), 3.10 (2H, d, *J* 7 Hz), 3.42 (1H, dd, *J* 17 and 3 Hz), 4.68 (1H, t, *J* 7 Hz), 4.7—4.9 (2H, broad m), 5.04 (1H, s), 5.16 (2H, s), 5.58—5.70 (3H, broad m), 7.32 (5H, s).

## Example 8

9-*N*-Hexahydrobenzylaminodeoxyclavulanic acid

Benzyl 9-*N*-(2″-methylallyl)-*N*-(1′,2′,3′,6′-tetrahydrobenzyl)aminodeoxyclavulanate (1.26 g; 2.9 mM) in ethanol (50 $cm^3$) was hydrogenolysed at 1.013 × $10^5$ Pa (1 atmosphere) for 40 minutes in the presence of palladium on carbon (10%) (400 mg) which had been prehydrogenated for 1½ hour. The catalyst was filtered off and washed with ethanol (20 $cm^3$) then with 50% aqueous ethanol (100 $cm^3$). This aqueous washing was evaporated to yield a white crystalline solid. This solid was slurried in cold ethanol and filtered off, washed with a few $cm^3$ of cold (0°) ethanol and dried to yield 0.41 g (48%) of the title compound. Rf ($SiO_2$/butanol-propan-2-ol-water; 7:7:6) = 0.6 (detection by aqueous potassium permanganate spray). $\nu$ (Nujol) 1805, 1692, 1610 (broad), 1590 (broad), $cm^{-1}$. 'H nuclear magnetic resonance showed that the cyclohexenyl C = C was only partially reduced. The mixed zwitterionic products were dissolved in 50% aqueous ethanol (40 $cm^3$) and hydrogenated in the presence of 110 mg palladium on carbon (10%) for 3 hours at 1.013 × $10^5$ Pa (1 atmosphere), filtered and the catalyst washed with aqueous ethanol (20 $cm^3$), the filtrate was evaporated to yield a white crystalline solid, ethanol was added (20 $cm^3$) and cooled (0°). The solid was filtered off and washed with cold ethanol, dried to yield 0.3 g (30%) of the title compound. $\nu$ (Nujol) 1805, 1695, 1610 (broad), 1305, 1190, 1120, 1080, 1070, 1050, 1020, 1008, 950, 900, 850, 810, 760 $cm^{-1}$. $\delta$ ($D_2O$) 0.6—2.0 (11H, broad m), 2.83 (2H, d, *J* 6 Hz), 3.07 (1H, d, *J* 17 Hz), 3.55 (1H, dd, *J* 17 and 3 Hz), 3.67 (2H, d, *J* 7 Hz), 4.75 (1H, t, *J* 7 Hz), 4.95 (1H, s), 5.73 (1H, d, *J* 3 Hz).

## Example 9

Benzyl 9-N-(2′-methylallyl)-N-(n-propyl)aminodeoxyclavulanate

To a solution of benzyl dichloroacetylclavulanate (8.0 g, 20 mmol) in dimethylformamide (100 ml) at −10°C was added N-(2′methylallyl)-N-(*n*-propyl)amine (4.3 g, 38 mmol) in dimethylformamide (15 ml). After 30 mins at −10°C the reaction mixture was poured into ethyl acetate and extracted with water (4 x). The organic phase was washed with brine, dried ($MgSO_4$) and evaporated to a yellow oil which was chromatographed on silica gel. The title ester was obtained on elution with petrol/ethyl acetate: 1/1 grading to 1/2.

Yield, as an oil, 4.2 g (55%).

*I.R.* ($CDCl_3$) 2950, 1800, 1745, 1695 and 900 $cm^{-1}$

*N.M.R.* ($CDCl_1$) 0.81 (3H, t, *J* 7 Hz), 1.41 (2H, sextet, *J* 7 Hz), 1.68 (3H, s), 2.23 (2H, t, *J* 7 Hz), 2.81 (2H, s), 2.96 (1H, d, *J* 17 Hz), 3.10 (2H, d, *J* 7 Hz), 3.44 (1H, dd, *J* 17 and 3 Hz), 4.69 (1H, bt, *J* 7 Hz), 4.80 (2H, bs), 5.05 (1H, s), 5.16 (2H, s,) 5.63 (1H, d, *J* 3 Hz), and 7.34 (5H, s).

## Example 10

9-N-(*n*-Propyl)aminodeoxyclavulanic acid

A mixture of tetrahydrofuran (150 ml) and water (15 ml) containing 10% Pd on carbon (1.6 g)

was hydrogenated at $1.013 \times 10^5$ Pa (1 atmosphere) for 20 mins. Benzyl 9-N-(2′-methylallyl)-N-(*n*-propyl)aminodeoxyclavulanate (3.9 g, 10.2 mmol) in tetrahydrofuran (15 ml) was added and the hydrogenation was continued for 40 mins. The catalyst was filtered off (celite) and the filter cake was washed which crystallised on trituration with *iso*-propanol. Yield = 0.190 g.

The filter-pad from above was now washed with 50% aqueous ethanol. Evaporation and trituration with *iso*-propanol afforded the title compound as a white solid (0.91 g). Total yield of title compound = 1.1 g (45%).

*I.R.* (KBr) 3540—3370, 2960, 1795, 1780, 1700 and 1615 $cm^{-1}$.

*N.M.R.* ($D_2O$) 0.90 (3H, t, *J* 7 Hz), 1.64 (2H, sextet, *J* 7 Hz), 2.94 (2H, t, *J* 7 Hz), 3.09 (1H, d, *J* Hz), 3.56 (1H, dd, *J* 17 and 3 Hz), 3.69 (2H, d, *J* 7 Hz), 4.78 (1H, bt, *J* 7 Hz), 4.97 (1H, s), and 5.75 (1H, d, *J* 3 Hz).

## Example 11

Benzyl 9-N-(*n*-butyl)-N-(2′-methyl-3′-phenylallyl)aminodeoxyclavulanate

Benzyl 9-0-dichloroacetylclavulanate (9.33 g; 23.3 mmol) in dry dimethylformamide (90 ml) was stirred and cooled to −10°C. N-(n-Butyl)-N-(2′-methyl-3′-phenylallyl)amine (9 g; 44.3 mmol) in dry dimethylformamide (90 ml) was then added dropwise. The solution was then stirred at −10°C for 30 minutes.

The solution was then poured into an ethyl acetate/water mixture and shaken. The two layers were then separated and the aqueous phase was extracted with more ethyl acetate. The combined organic phases were then washed with water, brine, dried ($MgSO_4$), filtered and evaporated under a reduced pressure to give a dark yellow oil. Column chromatography through silica-gel eluting with ethylacetate petroleum ether 1:2 gave the title compound as a light yellow oil in a 35% yield.

$\nu$ max ($CHCl_3$): 1800, 1750, 1720 and 1640 $cm^{-1}$.

$\delta$ ($CDCl_3$): 0.70→1.05 (3H, m) 1.06→1.68 (4H, m), 1.85 (3H, s), 2.19→2.51 (2H, m), 2.90 (1H, d, *J* 17 Hz), 2.97 (2H, s), 3.19 (2H, d, *J* 7 Hz), 3.39 (1H, dd, *J* 17 and 3 Hz), 4.75 (1H, t, *J* 7 Hz), 5.09 (1H, s), 5.17 (2H, s), 5.62 (1H, d, *J* 3 Hz), 6.38 (1H, br, s), and 7.02→7.54 (10H, m).

## Example 12

9-N-(*n*)-Butyl)-aminodeoxyclavulanic acid

Benzyl 9-N-(*n*-butyl)-N-(2-methyl-3-phenylallyl)aminodeoxyclavulanate (3.80 g, 8 mmole) in tetrahydrofuran (T.H.F.) (40 ml) was added to a prehydrogenated mixture of 10% palladium on charcoal (2 g) in 10% aqueous T.H.F. The mixture was then hydrogenated at $1.013 \times 10^5$ Pa (1 atmosphere) for 1 hour.

The catalyst was then filtered through a celite pad and the 'cake' washed with 10% aqueous T.H.F. and then with 50% aqueous ethanol. The filtrate was then evaporated to dryness under a reduced pressure and T.H.F. added. The white crystalline solid was then filtered off and from the spectral data obtained found to be the title compound in 30% yield.

$\nu$ max (KBr): 1800, 1784, 1695, and 1610 $cm^{-1}$.

$\delta$ ($D_2O$): 1.01 (3H, m), 1.57 (4H, m), 3.12 (2H, t, *J* Hz), 3.25 (1H, d, *J* 17 Hz), 3.75 (1H, dd, *J* 17 and 3 Hz), 3.83 (2H, d, *J* 7 Hz), 4.93 (1H, broad, t, *J* 7 Hz), 5.14 (1H, s), and 5.88 (1H, d, *J* 3 Hz).

## Example 13

Benzyl 9-*N*-(2′-methylallyl)-*N*-(3″-methylallyl)aminodeoxyclavulanate

Benzyl dichloroacetylclavulanate (7.4 g; 18.5 mM) in dry dimethylformamide (50 $cm^3$) at 0°C was treated with *N*-(2′-methylallyl)-*N*-(3″-methylallyl) amine (1.9 equivalents) and stirred at 0° for 45 minutes. The mixture was poured into ethyl acetate (200 $cm^3$) and washed with water ($6 \times 100$ $cm^3$) and saturated brine ($6 \times 100$ $cm^3$), dried (anhydrous magnesium sulphate) and evaporated to an oil. This oil was chromatographed on silica eluting with ethyl acetate-cyclohexane (1:2), fractions were collected Rf ($SiO_2$/ethyl acetate-cyclohexane; 1:2) = 0.5 (detection by aqueous potassium permanganate spray). Combined fractions were evaporated to yield the title compound an oil, yield = 2.7 g (37%). $\nu$ (film) 1805, 1750, 1700, 1450, 1380, 1305, 1230, 1175, 1120, 1080, 1042, 1015, 970, 895, 740, 700 $cm^{-1}$. $\delta$ ($CDCl_3$) 1.58—180 (6H, m), 2.81 (4H, broads), 2.95 (1H, d, *J* 17 Hz), 3.08 (2H, d, *J* 7 Hz), 3.42 (1H, dd, *J* 17 and 3 Hz), 4.68 (1H, t, *J* 7 Hz), 4.79 (2H, broad s), 5.03 (1H, s), 5.15 (2H, s), 5.36—5.56 (2H, broad m), 5.60 (1H, d, *J* 3 Hz), 7.32 (5H, s).

## Example 14

9-*N*-n-Butylaminodeoxyclavulanic Acid

Benzyl 9-*N*-(2′-methylallyl)-*N*-(3″-methylallyl) aminodeoxyclavulanate (1.37 g); in ethanol (30 $cm^3$) was hydrogenolysed at $1.013 \times 10^5$ Pa (1 atmosphere) in the presence of palladium on carbon 10% (0.5 g, prehydrogenated for 20 minutes) for 25 minutes. The catalyst was filtered off and washed with aqueous ethanol (50 $cm^3$). The filtrate was evaporated, ethanol added and cooled (0°). A white crystalline solid was filtered off and dried (354 mg). Thin layer chromatography on silica showed that this solid was a mixture of 9-*N*-n-butylaminodeoxyclavulanic acid and 9-*N*-isobutylaminodeoxyclavulanic acid, the former being present as the major constituent, and less polar Rf ($SiO_2$-butanol-

propan-2-ol-water: 7:7:6) = 0.53 than the iso derivative Rf = 0.50. The mixture was chromatographed on cellulose eluting with butanol-propan-2-ol- water; 8:8:1. Fractions were collected containing the title compound Rf = 0.53. Combined fractions were evaporated to a white crystalline solid. 25 mg. $\nu$ (Nujol) (2800—2100), 1805, 1690, 1600 (broad), 1300, 1182, 1110, 1080, 1067, 1045, 1015, 1005, 940, 910, 892, 865, 805, 755 $cm^{-1}$.

$\delta$ ($D_2O$) 0.65—1.05 (3H, m) 1.05—1.82 (4H, m), 2.96 (2H, t, *J* 7 Hz), 3.07 (1H, d, *J* 17 Hz), 3.57 (1H, dd, *J* 17 and 3 Hz), 3.68 (2H, d, *J* 7 Hz), 4.76 (1H, t, *J* 7 Hz), 4.95, (1H, s), 5.73 (1H, d, *J* 3 Hz).

### Example 15

Benzyl 9-*N*-(3′,4′,5′-trimethoxybenzyl)-N-(2″-methylallyl)aminodeoxyclavulanate

Benzyldichloroacetylclavulanate (5.49 g; 13.7 mM) in dry dimethylformamide (50 $cm^3$) at 0°C was treated with *N*-(3,4,5-trimethoxybenzyl) 2′-methylallylamine (1.9 equivalents) dropwise with stirring. The mixture was stirred at 0° for 2 hours then poured into ethylacetate (250 $cm^3$) and washed with water (5 x 150 $cm^3$) and saturated brine (5 x 150 $cm^3$), dried (anhydrous magnesium sulphate) and evaporated to an oil. This oil was chromatographed on silica eluting with ethylacetate-cyclohexane: 1:2. Fractions were collected containing material Rf 0.78 ($SiO_2$/ethylacetate-cyclohexane; 1:1). Combined fractions were evaporated to yield the title compound as an oil; yield = 2.4 g (34%). $\nu$ (film) 1805, 1750, 1695, 1590, 1502, 1455, 1420, 1330, 1308, 1185, 1175, 1125, 1010, 895, 745, 700 $cm^{-1}$. $\delta$ ($CDCl_3$) 1.73 (3H, s), 2.85 (2H, s) 2.96 (1H, d, *J* 17 Hz), 3.13 (2H, d, *J* 7 Hz), 3.37 (2H, s), 3.45 (1H, dd, *J* 17 and 3 Hz), 3.82 (9H, s), 4.73 (1H, t, *J* 7 Hz), 4.86 (2H, broad s), 5.07 (1H, s), 5.18 (2H, s), 5.61 (1H, d, *J* 3 Hz), 6.54 (2H, s), 7.31 (5H, s).

### Example 16

9-*N*-(3′,4′,5′-Trimethoxybenzyl)aminodeoxyclavulanic acid

Benzyl 9-*N*-(3′,4′,5′-trimethoxybenzyl)-*N*-(2″-methylallyl)aminodeoxyclavulanate (2.0 g; 3.83 mM) in ethanol (50 $cm^3$) was hydrogenolysed at 1.013 x $10^5$ Pa (1 atmosphere) in the presence of palladium on carbon (10%), 0.6 g (prehydrogenated for 15 minutes) for 20 minutes, water (10 $cm^3$) was added and hydrogenolysis continued for 10 minutes. The catalyst was filtered off and washed with aqueous ethanol (30 $cm^3$) and the clear filtrate evaporated. Ethanol (30 $cm^3$) was added to yield a white crystalline solid. The solid was filtered off cold (0°) and washed with a little cold ethanol. Drying gave the title compound as a finely crystalline solid, yield = 0.61 g (42%), Rf ($SiO_2$/butanol-propan-2-ol-water; 7:7:6) = 0.55.

$\nu$ (Nujol) 1805, 1695, 1615, 1595, 1300, 1250, 1192, 1165, 1130, 1045, 1008, 920, 895, 830, 782, 750 $cm^{-1}$, $\nu$ (KBr) 1790, 1695, (1620—1590), 1510, 1464, 1427, 1385, 1334, 1300, 1247, 1190, 1160, 1123, 1040, 1020, 1005, 957, 920, 895, 850, 830, 783, 745 $cm^{-1}$. $\delta$ ($D_2O$) 2.99 (1H, d, *J* 17 Hz), 3.50 (1H, dd, *J* 17 and 3 Hz), 3.64 (2H, d, *J* 7 Hz), 3.72 (3H, s, ), 3.80 (6H, s), 4.05 (2H, s), 4.74 (1H, t, *J* 7 Hz), 4.92 (1H, s), 5.64 (1H, d, *J*3 Hz), 6.72 (2H, s).

### Example 17

Benzyl 9,N,N-bis(2′-methylallyl)aminodeoxyclavulanate

Benzyl dichloroacetylclavulanate (5 g; 12.5 mm) in dry dimethylformamide (75 $cm^3$) at 0° was treated with bis (2-methylallyl)amine (1.9 equivalents) and stirred at 0° for 2 hours. The mixture was poured into ethylacetate (250 $cm^3$) and washed with water (5 x 100 $cm^3$) and saturated brine (5 x 100 $cm^3$), dried (anhydrous magnesium sulphate) and evaporated to an oil. This oil was chromatographed on silica eluting with ethylacetecyclohexane (1:1). Fractions were collected containing the title compound Rf ($SiO_2$/ethylacetate-cyclohexane; 1:1) = 0.82 (detection by aqueous potassium permanganate spray). Combined fractions were evaporated to yield an oil, 1.46 g (29%).

$\nu$ (film) 1805, 1750, 1695, 895, 755, 700 $cm^{-1}$. $\delta$ ($CDCl_3$) 1.67 (6H, s), 2.78 (4H, s), 2.95 (1H, d, *J* 17 Hz), 3.03 (2H, d, *J* 7 Hz), 3.42 (1H, dd, *J* 17 and 3 Hz), 4.67 (1H, t, *J* 7 Hz), 4.80 (4H, broads), 5.03 (1H, s), 5.27 (2H, s), 5.61 (1H, d, *J* 3 Hz), 7.33 (5H, s).

### Example 18

9-N-Isobutylaminodeoxyclavulanic acid

Benzyl 9-N,N-bis(2′-methylallyl)aminodeoxyclavulanate (0.8 g; 2.02 mm) in ethanol (30 $cm^3$) was hydrogenated in the presence of palladium on charcoal 10% (0.3 g) for 10 min at 1.013 x $10^5$ Pa (1 atmosphere) the catalyst had been pre-hydrogenated for 20 minutes. The catalyst was filtered off and washed with aqueous ethanol (50 $cm^3$), the filtrate was evaporated in vacuo and ethanol added, after cooling at 0° a crystalline solid was filtered off and washed with cold ethanol, drying in vacuo afforded the title compound as a white crystalline solid; yield = 190 mg (37%) Rf ($SiO_2$/butanol-propan-2-ol-water; 7:7:6) = 0.45 $\nu$ (Nujol) 1805, 1690, 1610, 1570, 1185, 1110, 1080, 1070, 1045, 1020, 1005, 930, 895, 885, 857, 810, 758 $cm^{-1}$. $\nu$ (KBr) 3570 (broad), 3350 (broad), 3220 (broad), 2840—2740, 2450 (broad), 1780, 1695, 1600 (very broad), 1470, 1392, 1320, 1295, 1110, 1045, 1020, 1003, 987, 932, 893, 885, 812, 750 $cm^{-1}$. $\delta$ ($D_2O$) 0.94 (6H, d, *J*, 6 Hz), 1.90 (1H, m), 2.82 (2H, d, *J* 6 Hz), 3.07 (1H, d, *J* 17 Hz), 3.57 (1H, dd, *J* 17 and 3 Hz), 3.70 (2H, d, *J* 17 and 3 Hz), 3.70 (2H, d, *J* 7 Hz), 4.77 (1H, t, *J* 7 Hz), 4.96 (1H, d), 5.73 (1H, d, *J* 3 Hz).

Example 19

Benzyl 9-N-isobutyl-N-(2′-methyl-3′-phenylallyl)aminodeoxyclavulanate

Benzyl dichloroacetylclavulanate (7.16 g; 17.9 mM) in dry dimethylformamide (85 $cm^3$) at −12° was treated with 1.9 equivalents of *N*-isobutyl-*N*-(2-methyl-3-phenylallyl) amine and stirred at −15° for 15 minutes then for 45 minutes between −10° and 0°. The mixture was poured into iced ethylacetate (250 $cm^3$) and washed with water (5 x 100 $cm^3$) saturated brine (5 x 100 $cm^3$), dried (anhydrous magnesium sulphate) and evaporated in the presence of toluene to a small volume. This crude product was chromatographed on silica eluting with ethylacetate-cyclohexane; 1:2. Fractions were collected containing the title compound Rf ($SiO_2$/ethylacetate-cyclohexane; 1:2) = 0.89. Combined fractions were evaporated to afford the title compound as oil, yield = 1.60 g (19%), $\nu$ (film) 1808, 1750, 1690, 745, 700 $cm^{-1}$. $\delta$ ($CDCl_3$) 1.85 (6H, d, *J* 6 Hz), 1.50—2.20 (6H, broad m), 2.92 (1H, d, *J* 17 Hz), 2.94 (2H, s), 3.39 (1H, dd, *J* 17 and 3 Hz), 4.73 (1H, broad, t, *J* 7 Hz), 5.07 (1H, broad s), 5.17 (2H, s), 5.60 (1H, d, *J* 3 Hz), 6.35 (1H, broad s), 7.25 and 7.32 (10 H, 2 x s).

Example 20

9-*N*-Isobutylaminodeoxyclavulanic acid

Benzyl 9-*N*-(2′-methyl-3′-phenylallyl)-*N*-isobutylaminodeoxyclavulanate (1.44 g; 3.04 mM) in ethanol (25 $cm^3$) was hydrogenolysed in the presence of palladium on carbon (10% Pd), 0.5 g (which had been prehydrogenated for 15 minutes), for 45 minutes at 1.013 x $10^5$ Pa (1 atmosphere). The catalyst was filtered off and washed with ethanol (50 $cm^3$), then with aqueous ethanol (100 $cm^3$), the aqueous washing was collected separately and was evaporated to yield the title compound as a white crystalline solid. The solid was washed with a little cold ethanol, drying afforded 248 mg of the title compound. The ethanolic catalyst washing and the solvent from the hydrogenolysis were evaporated, ethanol added (10 $cm^3$) and cooled, crystals were filtered off and dried to yield a further 17 mg of the title compound, total yield = 265 mg (34%). The infrared and proton magnetic resonance spectra were identical to the product obtained by hydrogenolysis of benzyl 9-*N*-N-bis(2′-methylallyl)aminodeoxyclavulanate.

Example 21

Benzyl 9-*N*-(2′-methylallyl)-*N*-(3″-methylallyl)aminodeoxyclavulanate

Benzyl dichloroacetylclavulanate (7.4 g; 18.5 mM) in dry dimethylformamide (50 $cm^3$) at 0° was treated with *N*-(2′-methylallyl)-*N*-(3″-methylallyl) amine (1.9 equivalents) and stirred at 0° for 45 minutes. The mixture was poured into ethyl acetate (200 $cm^3$) and washed with water (6 x 100 $cm^3$) and saturated brine (6 x 100 $cm^3$), dried (anhydrous magnesium sulphate) and evaporated to an oil. This oil was chromatographed on silica eluting with ethyl acetate-cyclohexane (1:2), fractions were collected Rf ($SiO_2$/ethyl acetate-cyclohexane; 1:2) = 0.5 (detection by aqueous potassium permanganate spray). Combined fractions were evaporated to yield the title compound as an oil, yield = 2.7 g (37%). $\nu$ (film) 1805, 1750, 1700, 1450, 1380, 1305, 1230, 1175, 1120, 1080, 1042, 1015, 970, 895, 740, 700 $cm^{-1}$.

$\delta$ ($CDCl_3$) 1.58—1.80 (6H, m), 2.81 (4H, broads), 2.95 (1H, d, *J* 17 Hz), 3.08 (2H, d, *J* 7 Hz), 3.42 (1H, dd, *J* 17 and 3 Hz), 4.68 (1H, t, *J* 7 Hz), 4.79 (2H, broad s), 5.03 (1H, s), 5.15 (2H, s), 5.36—5.56 (2H, broad m), 5.60 (1H, d, *J* 3 Hz), 7.32 (5H, s).

Example 22

9-*N*-Isobutylaminodeoxyclavulanic acid

Benzyl 9-*N*-(2′-methylallyl)-N-(3′-methylallyl)aminodeoxyclavulanate (137 mg) in ethanol (30 $cm^3$) was hydrogenolysed at 1.013 x $10^5$ Pa (1 atmosphere) in the presence of 10% palladium on charcoal (0.5 g which had been prehydrogenated for 20 minutes) for 25 minutes. The catalyst was filtered off and washed with aqueous ethanol, the filtrate was evaporated and ethanol added. The resulting crystals were filtered off cold and dried. This product was chromatographed on cellulose eluting with butanol-isopropanol-water; 4:4:1. Fractions were collected containing the title compound in low yield Rf($SiO_2$/butanol-isopropanol water; 7:7:6) = 0.46 (detection by aqueous potassium permanganate spray).

Example 23

Benzyl 9-*N*-(2′-methylallyl)-*N*-(3″-transphenylallyl)aminodeoxyclavulanate

Benzyl dichloroacetylclavulanate (5.9 g; 14.8 mM) in dimethylformamide (40 $cm^3$) at 0° was treated with *N*-(2′-methylallyl)-*N*-(3′-*trans*phenylallyl) amine (1.9 equivalents) and stirred at 0° for 1$\frac{3}{4}$ hours. The mixture was then poured into ethyl acetate (250 $cm^3$) and washed with water (5 x 100 $cm^3$) and saturated brine (5 x 100 $cm^3$), dried (anhydrous magnesium sulphate) and evaporated to an oil. This oil was chromatographed on silica eluting with ethylacetate-cyclohexane (1:2), fractions were collected containing the title compound; Rf($SiO_2$/ethylacetate:cyclohexane 1:1) = 0.76 and combined fractions were evaporated to yield an oil, 3.49 g (51%). Detection by aqueous potassium permanganate spray. $\nu$ (film) 1805, 1750, 1700, 1495, 1450, 1305, 1230, 1175, 1120, 1080, 1045, 1015, 967, 895, 745, 695 $cm^{-1}$. $\delta$ ($CDCl_3$) 1.71 (3H, s), 2.89 (2H, s), 2.93 (1H, d, *J* 17 Hz), 3.07 (2H, d, *J* 6 Hz),

3.16 (2H, d, $J$ 7 Hz), 3.39 (1H, dd, $J$ 17 and 3 Hz), 4.72 (1H, t, $J$ 7 Hz), 4.75—4.95 (2H, broad m), 5.06 (1H, d, $J$ 3 Hz), 5.16 (2H, s), 6.15 (1H, dt, $J$ 16 and 6 Hz), 6.45 (1H, d, $J$ 16 Hz), 7.15—7.50 (10H, m).

Example 24

9-*N*-Isobutylaminodeoxyclavulanic acid

Benzyl 9-*N*-(2′-methylallyl)-*N*-(3″-*trans* phenylallyl) aminodeoxyclavulanate (11 g) in ethanol (40 $cm^3$) was hydrogenolysed for 20 minutes at 1.013 × $10^5$ Pa (1 atmosphere) in the presence of 10% palladium on charcoal (300 mg; which had been prehydrogenated for 10 minutes). The catalyst was filtered off and washed with aqueous ethanol. The filtrate was evaporated and ethanol added, the resulting crystals were filtered off cold and dried. This product was chromatographed on cellulose eluting with butanol-isopropanol water; 4:4:1. Fractions were collected containing the title compound in low yield Rf($SiO_2$/butanol-isopropanol-water; 7:7:6) = 0.46 (detection by aqueous potassium permanganate spray).

Example 25

Benzyl 9-N(2′-methylallyl)-N-(2″-methyl-3″-phenylallyl)aminodeoxyclavulanate

Benzyl 9-O-dichloroacetylclavulanate (6.30 g; 15.8 mmole) in acetonitrile (60 ml) at 4°C was treated with dropwise addition of N-(23-methylallyl)-N-(2″-methyl-3″-phenylally) amine (6 g; 30 mmol) in acetonitrile (60 ml). On final addition the reaction was stirred at 4 to 10°C for 2 hours. The acetonitrile was then removed under a reduced pressure and the resulting oil was dissolved in ethyl acetate. The solution was then washed with water, brine, dried ($MgSO_4$) and evaporated *in vacuo*. Silica-gel column chromatography afforded the title compound as an oil in a 16% yield.

$\nu$ max ($CHCl_3$): 1802, 1745, 1695 and 1600 (br) $cm^{-1}$.

$\delta$ ($CDCl_3$): 1.70 (3H, s), 1.82 (3H, s), 2.84 (2H, s), 2.92 (2H, s), 2.90 (1H, d, $J$ 17 Hz), 3.12 (2H, d, $J$ 7 Hz), 3.38 (1H, dd, $J$ 17 and 3 Hz), 4.70 (1H, br.t, $J$ 7 Hz), 4.82 (2H, br.s), 5.06 (1H, s), 5.16 (2H, s), 5.60 (1H, d, $J$ 3 Hz), 6.36 (1H, s), 7.22 and 7.30 (10H, 2 x s).

Example 26

9-N-Isobutylaminodeoxyclavulanic acid

Benzyl-9-N-(2′-methylallyl)-N-(2″-methyl-3″phenylallyl) aminodeoxyclavulanate (1.0 g; 2 mmol) in ethanol (20 ml) was carefully added to a pre-hydrogenated mixture of 10% palladium on charcoal (300 mg) in ethanol (30 ml). The mixture was then hydrogenated at 1.013 × $10^5$ Pa (1 atmosphere) for 30 minutes. The catalyst was then filtered off and washed well with aqueous ethanol. The filtrate plus washings were then evaporated to dryness under a reduced pressure. Cellulose column chromatography afforded the title compound as a white crystalline solid in 30% yield.

Example 27

Benzyl 9-*N*-(*iso*-butyl)-*N*-(2′-methylallyl)aminodeoxyclavulanate

*N*-Isobutyl-*N*-(2-methylallyl)amine (7.25 g, 57 mmol) in dimethylformamide (30 ml) was added dropwise to a solution of benzyl dichloracetylclavulanate (12.0 g, 30 mmol) in dimethylformamide (200 ml) at −10°C. After 2 hours at this temperature the reaction mixture was poured into water and extracted with ethylacetate. The organic phase was washed several times with brine, dried ($MgSO_4$) and evaporated to a yellow oil. Chromatography in silica gel (elution: petrol/ethylacetate: 3/1 grading to 2/1) afforded the title ester as an oil, 2.98 g (25%).

I.R. ($CHCl_3$) 1802, 1745, 1700, and 895 $cm^{-1}$.

N.M.R. ($CDCl_3$) 0.84 (6H, d, $J$ 7 Hz), 1.52—1.88 (1H, m) 1.68 (3H, s), 2.00 (2H, d, $J$ 7 Hz), 2.79 (2H, s), 2.94 (1H, d, $J$ 17 Hz), 3.06 (2H, d, $J$ 8 Hz), 3.41 (1H, dd, $J$ 17 and 3 Hz), 4.68 (1H, bt, $J$ 8 Hz), 4.78 (2H, bs), 5.03 (1H, bs), 5.17 (2H, s), 5.61 (1H, d, $J$ 3 Hz), and 7.32 (5H, s).

Example 28

9-*N*-Isobutylaminodeoxyclavulanic acid

10% Pd-C (0.83 g) in ethanol (80 ml) was hydrogenated for 15 minutes at 1.013 × $10^5$ Pa (1 atmosphere). Benzyl 9-*N*-(isobutyl)-*N*-(2′-methylallyl)aminodeoxyclavulanate (2.5 g, 6.28 mmol) in ethanol (30 ml) was added and the hydrogenation continued for 45 minutes. The catalyst was then filtered off through celite and the pad was washed with some ethanol. These combined washings were evaporated to a yellow oil which crystallised from ethanol (0.120 g).

The filter pad, above was now washed with 50% aqueous ethanol (150 ml). Evaporation afforded the title material as a white solid (0.70 g).

Combination of all the mother liquors and evaporation afforded a dark oil which was chromatographed on silica gel (elution: ethylacetate/isopropanol/water: 5/2/1 grading to 5/4/3) to provide more of the title product (0.115 g).

Total yield of required product = 0.935 g (59% yield).

N.M.R., I.R., and T.L.C. characteristics were identical to an authentic sample.

## Example 29

Phenacyl 9-N-Bis (2′-methylallyl)aminodeoxyclavulanate

Phenacyl (9-0-dichloroacetylclavulanate (5.0 g; 11.7 mmol) in dry dimethylformamide (50 ml). The mixture was cooled to 0°C and bis (2′-methylallyl) amine (2.8 g; 22.2 mmol) in dry dimethylformamide (30 ml) was added dropwise. After stirring at 0°C for 1½ hours the mixture was poured into ethyl acetate and washed several times with water and then dried ($MgSO_4$). After filtration the ethyl acetate was removed *in vacuo* to give a yellow oil, which on column chromatography afforded the required compound as a yellow gum, yield 32%.

$[\alpha]_D^{20}$ + 11.0° (c. 1%, $CHCl_3$). Found C, 67.60; H, 6.70; N 6.38%, $C_{24}H_{28}H_2O_5$ requires: C, 67.91; H, 6.65; N, 6.60% $\nu_{max}$ ($CHCl_3$) 1805, 1760, and 1605 $cm^{-1}$; δ ($CDCl_3$) 1.72 (6H, s), 2.76 → 3.22 (7H, m), 3.45 (1H, dd, *J* 17 and 3 Hz), 4.82 (5H, m), 5.19 (1H, s), 5.38 (2H, s), 5.66 (1H, d, *J* 3 Hz), 7.30 → 7.60 and 7.78→8.00.

## Example 30

Allyl 9-*N,N*-bis(2′-methylallyl)aminoclavulanate

Allyl dichloroacetylclavulanate (5 g; 14.3 mM) in dry dimethylformamide (50 $cm^3$) at 0° was treated with bis (2 methylallyl)amine (1.9 equivalents) and stirred 2½ hours at between 0° and 5°C. The mixture was poured into ethylacetate (250 $cm^3$) and washed with water (5 x 200 $cm^3$) and saturated brine (5 x 150 $cm^3$), dried (anhydrous magnesium sulphate) and evaporated to an oil. This oil was chromatographed on silica eluting with ethylacetate-cyclohexane; 1:1. Fractions were collected containing the title compound, Rf ($SiO_2$/ethylacetate-cyclohexane; 1:1) = 0.90 (detection by aqueous potassium permanganate spray). Combined fractions were evaporated *in vacuo* to yield an oil, 1.33 g (27%), (film) 1808, 1750, 1695, 1450, 1370, 1308, 1232, 1180, 1120, 1015, 940, 895 $cm^{-1}$.

($CDCl_3$) 1.71 (6H, s), 2.83 (4H, s), 2.98 (1H, d, *J* 17 Hz), 3.07 (2H, d, *J* 7 Hz), 3.45 (1H, dd, *J* 17 and 3 Hz), 4.55—4.94 (7H, m), 5.02 (1H, s), 5.17—5.5 (2H, m), 5.63 (1H, d, *J* 3 Hz), 5.6—6.16 (1H, m).

## Example 31

(2-Methylallyl) 9′-N-(Isobutyl)-N-(2″-Methylallyl) aminodeoxyclavulanate

(2-Methylallyl)-9′-0-dichloroacetylclavulanate (9.05 g; 24.8 mmol) in anhydrous dimethylformamide (90 ml) was cooled with stirring to −20°C. N-(Isobutyl)-N-(2-methylallyl) amine (6.0 g; 47 mmol) in anhydros dimethylformamide (60 ml) was added dropwise over a period of 20 minutes. The reaction mixture was allowed to warm to −10°C and stirring continued for 2 hours.

The solution was poured into a cold mixture of ethyl acetate and water and shaken. The aqueous layer was extracted with more ethyl acetate. The combined organic layers were washed with water, dried ($MgSO_4$) and evaporated to an oil. Column chromatography afforded the title compound as a colourless oil in 23% yield.

$\nu_{max}$ ($CHCl_3$): 1805, 1750 and 1700 $cm^{-1}$. δ ($CDCl_3$): 0.85 (6H, d, *J* 7 Hz), 1.70 (3H, s), 1.77 (3H, s), 1.57 to 1.88 (1H, m), 2.05 (2H, d, *J* 7 Hz), 2.84 (2H, s), 2.94 (1H, d, *J* 17 Hz), 3.11 (2H, d, *J* 7 Hz), 3.46 (1H, dd, *J* 17 and 3 Hz), 4.58 (2H, s), 4.74 (1H, broad t, *J* 7 Hz), 4.81 (2H, broad s), 4.99 (2H, broad s), 5.06 (1H, s), and 5.66 (1H, d, *J* 3 Hz).

## Example 32

9-N-Isobutylaminodeoxyclavulanate

(2-Methylallyl)-9′-N-(isobutyl)-N-(2″-methylallyl) aminodeoxyclavulanate (1.5 g; 4 mmol) in ethanol (20 ml) was carefully added to a pre-hydrogenated mixture of 10% palladium on charcoal (0.5 g) in ethanol (20 ml). The mixture was hydrogenated at 1.013 x $10^5$ Pa (1 atmosphere) until the uptake of hydrogen ceased.

The mixture was filtered through a celite pad and the "cake" washed with aqueous ethanol. The filtrate plus washings were evaporated to dryness resulting in a yellow solid which an addition of a small amount of ethanol gave a white solid. Filtration afforded the title compound in 20% yield. Spectral data was consistent with an authentic example.

## Example 33

Benzyl 9-*N*-methyl-*N*-(2′-methylallyl)aminodeoxyclavulanate.

A solution of benzyl dichloroacetylclavulanate (6 g; 0.015 mol) in dimethylformamide (80 ml) was cooled to −20° and a solution of *N*-methyl-*N*-(2-methylallyl)amine (2.4 g; 0.028 mol) in dimethylformamide (10 ml) was added slowly dropwise over 10 minutes. The reaction mixture was stirred at −10° for 2 hours then poured into ethyl acetate, washed with water, brine, dried and evaporated. Two crude product was chromatographed on silica eluting with ethyl acetate-cyclohexane, 3:1. Combined fractions were evaporated to afford the title compound as an oil, yield = 9%.

$\nu_{max}$ ($CHCl_3$) 1795, 1730, 1690 (sh), 1630 $cm^{-1}$.

δ ($CDCl_3$) 1.71 (3H, s), 2.09 (3H, s) 2.81 (2H, s), 2.98 (1H, d), 3.04 (2H, d, *J* 8 Hz), 3.45 (1H, d, *J* 3 Hz), 4.72 (1H, bt, *J* 8 Hz), 4.82 (2H, bs), 5.07 (1H, bs), 5.18 (2H, s), 5.63 (1H, d, *J* 3 Hz), 7.3 (5H, s).

Example 34

9-$N$-Methylaminodeoxyclavulanic acid

A solution of benzyl 9-$N$-methyl-$N$-(2′-methylallyl) amindeoxyclavulanate (200 mg) in ethanol: tetrahydrofuran: water (7:2:1, 30 ml) was hydrogenolysed at $1.013 \times 10^5$ Pa (1 atmosphere) in the presence of 10% palladium on carbon (70 mg) for 2 hours. The catalyst was filtered off and the pad washed well with aqueous ethanol. The filtrate and washings were combined and evaporated to give a solid which crystallised from acetone to afford the title compound in 40% yield. This product was identical (t.l.c., i.r., n.m.r) to an authentic sample of 9-$N$-methylaminodeoxyclavulanic acid.

Example 35

Benzyl 9-$N$-methyl-$N$-(2′-methyl-3′-phenylallyl)aminodeoxyclavulanate

A cold (−10°) solution of benzyl dichloroacetylclavulanate (6.5 g; in 0.016 mol) in dimethylformamide (75 ml) was treated dropwise with a solution of $N$-methyl-$N$-(2-methyl-3-phenylallyl) amine in dimethylformamide (25 ml) and stirred for 4 hours at −10°. The mixture was poured into ethyl acetate (150 ml) and extracted with aqueous tartaric acid (3.0 g in 100 ml water: 0.02 mol), the acid extract was basified to pH 8.3 and re-extracted with ethyl acetate. The ethyl acetate solution was washed with brine, dried (di-sodium hydrogen orthophosphate) and evaporated. Toluene was added during evaporation until only a small amount of solvent was present, this was loaded onto a silica gel column and the product eluted with ethyl acetate. The product was isolated as an oil in 7% yield.

$\nu_{max}$ (film), 1805, 1750, 1700 $cm^{-1}$.

$\delta$ ($CDCl_3$) 1.85 (3H, d, $J$ 1.5 Hz), 2.13 (3H, s), 2.93 (1H, d, $J$ 17 Hz), 2.92 (2H, s), 3.09 (2H, d, $J$ 8 Hz), 3.39 (1H, dd, $J$ 17 and 3 Hz), 4.74 (1H, dt, $J$ 8 and 1.5 Hz), 5.07 (1H, bs), 5.16 (2H, s), 5.61 (1H, d, $J$ 3 Hz), 6.36 (1H, bs), 7.25, 7.31 (10H, 2xs). Found $M^+$ 432.2069, $C_{26}H_{28}N_2O_4$ requires 432.2049.

Example 36

9-$N$-methylaminodeoxyclavulanic acid

Benzyl 9-$N$-methyl-$N$-(2′-methyl-3′-phenylallyl)aminodeoxyclavulanate (500 mg) in ethanol (50 ml) was hydrogenolysed at $1.013 \times 10^5$ Pa (1 atmosphere) in the presence of 10% Pd—C (160 mg) for 3 hours. The catalyst was filtered off and washed with aqueous ethanol; the filtrate and washings were combined and evaporated. The crude product was fractionated on silica gel using ethyl acetate: isopropanol: water, 5:4:4, as eluent.

The secondary amine was eluted and isolated as a crystalline solid in 20% yield.

(KBr) 3420, 3000, 2760, 2430, 1779, 1693, 1618 $cm^{-1}$.

$\delta$ ($D_2O$) 2.65 (3H, s), 3.15 (1H, d, $J$ 17 Hz), 3.62 (1H, dd, $J$ 17 and 3 Hz), 3.74 (2H, d, $J$ 8 Hz), 4.85 (1H, bt, $J$ 8 Hz), 5.05 (1H, bs), 5.82 (1H, d, $J$ 3 Hz).

Description 1

Allyl 9-0-dichloroacetylclavulanate

Allylclavulanate (6.3 g; 26 mm) in dichloromethane (100 $cm^3$) at −30°C was treated with pyridine (3 $cm^3$) and dichloroacetylchloride (1 equivalent) and stirred for 10 minutes at −20°C. The mixture was diluted with dichloromethane (100 $cm^3$) and washed with aqueous citric acid solution (10%; 50 $cm^3$), water (3 × 100 $cm^3$), saturated brine (3 × 150 $cm^3$), dried (anhydrous magnesium sulphate) and evaporated to an oil yield = 8.3 g (90%), Rf ($SiO_2$/ethylacetate-cyclohexane 1:1) =0.78.

Description 2

Phenacyl-9-Dichloroacetyl Clavulanate

Phenacyl clavulanate (14.26 g; 45 mmol) in dry methylene chloride (100 ml) was treated with pyridine (4 mls, 50.6 mmol) at room temperature. The reaction mixture was cooled to −30°C and dichloroacetyl chloride (4.3 mls, 1.2 equivalents) in dry methylene chloride (15 ml) was added dropwise. After stirring at −30°C for 30 minutes the mixture was allowed to warm to 0°C and was then poured into dilute hydrochloric acid. The organic phase was separated and washed with more dilute hydrochloric acid, sodium bicarbonate solution, water, brine and then dried ($MgSO_4$). After filtration the methylene chloride was removed *in vacuo* to afford a yellow oil which crystallised on trituration with ether. Yield = 11.21 g, 50%. A sample recrystallised from ether gave the following physical characteristics:

m.p. 75—77°C $[\alpha]_D^{20}$ + 16.1°, (c. 1%; $CHCl_3$).

Description 3

(2-Methylallyl)clavulanate

Sodium clavulanate (25 g; 83 mmol) in anhydrous dimethylformamide (100 ml) was treated at room temperature over a period of 15 minutes with methallychloride (15.1 g; 166 mmol) in dimethylformamide (50 ml) plus a catalytic amount of sodium iodide. The mixture was then stirred at room temperature overnight.

The mixture was then poured into an ethyl acetate/water mixture and shaken. The two layers

were separated and the aqueous layer extracted with ethyl acetate. The organic layers were combined, washed with water, washed with a saturated solution of sodium chloride, dried ($MgSO_4$) and evaporated to give the title compound as an oil in 73% yield.

$\nu_{max}$ ($CHCl_3$): 1810, 1750, 1695 and 1660 $cm^{-1}$.

($CDCl_3$): 1.75 (4H, s), 3.05 (1H, d, $J$ 17 Hz), 3.50 (1H, dd, $J$ 17 and 3 Hz), 4.23 (2H, d, $J$ 7 Hz), 4.58 (2H, s), 4.93 (1H, t, $J$ 7 Hz), 5.00 (2H, broad s), 5.09 (1H, m), and 5.70 (1H, d, $J$ 3 Hz).

Description 4
(2-Methylallyl)-9'-0-Dichloroacetylclavulanate

(2-Methylallyl)clavulanate (15.3 g; 60 mmole) in anhydrous dichloromethane (150 ml) was treated at room temperature with anhydrous pyridine (4.8 ml; 60 mmol) and was added dropwide over a period of 10 minutes. The reaction mixture was stirred at −40°C for 1 hour and then allowed to warm to 0°C.

The mixture was poured into cold dilute hydrochloric acid and separated. The organic layer was then washed several times with more dilute hydrochloric acid, water and a saturated solution of sodium chloride, dried ($MgSO_4$) and evaporated to an oil affording the title compound in 87% yield.

$\nu_{max}$ ($CHCl_3$): 1810, 1755 and 1700 $cm^{-1}$.

($CDCl_3$): 1.76 (3H, s), 3.10 (1H, d, $J$ 17 Hz), 3.56 (1H, dd, $J$ 17 and 3 Hz), 4.60 (2H, s), 4.78 to 5.00 (5H, m), 5.13 (1H, s), 5.77 (1H, d, $J$ 3 Hz), and 5.93 (1H, s).

**Claims for the Contracting States: BE CH DE FR GB IT LU NL SE**

1. A process for the preparation of a compound of the formula (I):

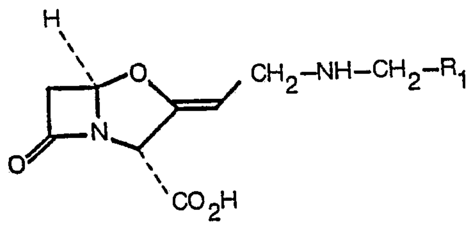

wherein $R_1$ is a hydrogen atom, an alkyl group of up to 5 carbon atoms, a cycloalkyl group of 5 or 6 carbon atoms, a hydroxyalkyl group of up to 5 carbon atoms or a moiety of sub formula (a):

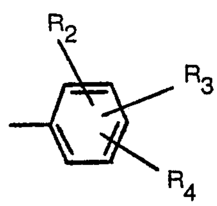

(a)

wherein $R_2$ is a hydrogen, fluorine, chlorine or bromine atom or an alkyl group of 1—3 carbon atoms, an alkoxyl group of 1—3 carbon atoms, an acyloxyl group of 1—3 atoms, a hydroxyl group, an alkoxycarbonyl group containing 1—3 carbon atoms in the alkoxy part, or a group $—N(R_5)CO.R_6$, $—N(R_5)SO_2R_6$ or $—CO—NR_5R_6$ where $R_5$ is a hydrogen atom or an alkyl group of 1—3 carbon atoms or a phenyl or benzyl group and $R_6$ is an alkyl group of 1—3 carbon atoms or a phenyl or benzyl group; $R_3$ is a hydrogen, fluroine or chlorine atom or an alkyl group of 1—3 carbon atoms, an alkoxyl group of 1—3 carbon atoms or an acyloxyl group of 1—3 carbon atoms; and $R_4$ is a hydrogen fluorine or chlorine atom or an alkyl group of 1—3 carbon atoms or an alkoxyl group which process comprises the catalytic hydrogenation of a compound of the formula (III):

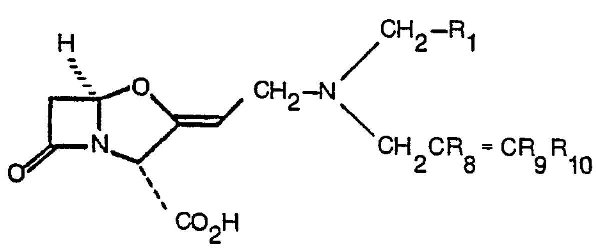

or a hydrogenolysable ester thereof wherein $R_1$ is as defined in relation to formula (I), or $R_1$ is $R_1^1$ where $R_1^1$ is a moiety that on hydrogenation provides a group $R_1$ as defined in relation to formula (I), $R_8$ is a

hydrogen atom or a lower alkyl group, $R_9$ is a hydrogen atom or a lower alkyl group and $R_{10}$ is a hydrogen atom or a lower alkyl or phenyl group; wherein "lower alkyl" means an alkyl group of up to 4 carbon atoms.

2. A process as claimed in claim 1 for the preparation of a compound of the formula (I) wherein $R_1$ is a phenyl group.

3. A process as claimed in claim 1 for the preparation of a compound of the formula (I) wherein $R_1$ is a p-acetamidophenyl group.

4. A process as claimed in claim 1 for the preparation of a compound of the formula (I) wherein $CH_2R_1$ is an isobutyl group which process comprises the hydrogenation of a compound of the formula (III) wherein $CH_2R_1{}^1$ is an isobutenyl group.

5. A process as claimed in claim 1 for the preparation of a compound of the formula (I) wherein $CH_2R_1$ is an isobutyl group which process comprises the hydrogenation of a compound of the formula (III) wherein $CH_2R_1$ is an isobutyl group.

6. A process as claimed in any of the claims 1 to 5 wherein the moiety $CH_2CR_8{=}CR_9R_{10}$ is $CH_2C(CH_3){=}CH_2$, $CH_2C(C_2H_5){=}CH_2$, $CH_2C(nC_3H_7){=}CH_2$, $CH_2C(CH_3){=}CHCH_3$, $CH_2C(CH_3){=}C(CH_3)_2$, $CH_2C(CH_3){=}CHC_2H_5$ or $CH_2C(CH_3){=}CH.C_6H_5$.

7. A process according to any of claims 1 to 5 where $CH_2CR_8 = CR_9R_{10}$ is $CH_2C(CH_3){=}CH_2$ or $CH_2C(CH_3) = CHC_6H_5$.

8. A process as claimed in any of claims 1 to 7 wherein the esterifying radical is benzyl, p-bromobenzyl, p-chlorobenzyl, p-methylbenzyl or p-methoxybenzyl.

9. A process as claimed in any of claims 1 to 7 wherein the esterifying radical is 2-methylallyl, 2-ethylallyl, 2-n-propylallyl, 2-methyl-3-methylallyl, 2-methyl-3,3-dimethylallyl, 2-methyl-3-ethylallyl or 2-methyl-3-phenylallyl.

10. A process as claimed in claim 1 for the preparation of 9-*N*-isobutylaminodeoxyclavulanic acid which comprises the hydrogenation of benzyl 9-*N*-(isobutyl)-*N*-(2′-methylallyl)aminodeoxyclavulanate.

11. A process as claimed in claim 1 for the preparation of 9-*N*-isobutylaminodeoxyclavulanic acid which comprises the hydrogenation of benzyl 9-*N*,*N*-bis(2′-methylallyl)aminodeoxyclavulanate.

12. A process as claimed in any of claims 1 to 11 wherein the hydrogenation reaction is performed in the presence of a palladium metal catalyst.

13. A process as claimed in claim 12 wherein the palladium catalyst is palladium on charcoal, palladium on barium sulphate, palladium on calcium carbonate or palladium black.

14. A process as claimed in claim 13 wherein the catalyst is palladium on carbon with a proportion of palladium between 1% and 30%.

15. A process as claimed in claim 14 wherein the proportion of palladium is 10%.

16. A process as claimed in any of claims 1 to 15 wherein the pressure of hydrogen used is between $1.013 \times 10^5$ Pa (1 atmosphere) and $6.09 \times 10^5$ Pa (6 atmospheres).

17. A process as claimed in any of claims 1 to 16 wherein the pressure of hydrogen is $1.013 \times 10^5$ Pa (1 atmosphere).

18. A process as claimed in any of claims 1 to 17 wherein the reaction temperature is maintained between 0° and 30°C.

19. A process as claimed in any of claims 1 to 18 wherein the solvent for the hydrogenation reaction is ethanol, n-propanol, isopropanol, tetrahydrofuran, dioxan or ethyl acetate, or a mixture of these solvents in the presence of water.

20. A process as claimed in claim 19 wherein the solvent is ethanol.

21. A process as claimed in claim 19 wherein the solvent is isopropanol.

22. A compound of the formula (III) or hydrogenolysable ester thereof wherein $R_1$, $R_9$ and $R_{10}$ are as defined in any one of claims 1 to 9 and $R_8$ is an alkyl group of up to 4 carbon atoms.

23. Benzyl 9-*N*-(isobutyl)-*N*-(2′-methylallyl)aminodeoxycalvulanate.

24. Benzyl 9-*N*,*N*-bis(2′-methylallyl)aminodeoxyclavulanate.

**Claims for the Contracting State: AT**

1. A process for the preparation of a compound of the formula (I):

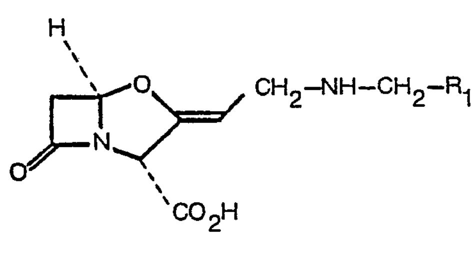

wherein $R_1$ is a hydrogen atom, an alkyl group of up to 5 carbon atoms, a cycloalkyl group of 5 or 6 carbon atoms, a hydroxyalkyl group of up to 5 carbon atoms or a moiety of sub formula (a):

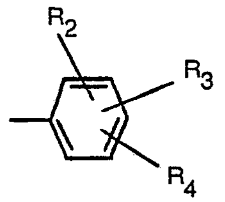

(a)

wherein $R_2$ is a hydrogen, fluorine, chlorine or bromine atom or an alkyl group of 1—3 carbon atoms, an alkoxyl group of 1—3 carbon atoms, an acyloxyl group of 1—3 atoms, a hydroxyl group, an alkoxycarbonyl group containing 1—3 carbon atoms in the alkoxy part, or a group $—N(R_5)CO.R_6$, $—N(R_5)SO_2R_6$ or $—CO—NR_5R_6$ where $R_5$ is a hydrogen atom or an alkyl group of 1—3 carbon atoms or a phenyl or benzyl group and $R_6$ is an alkyl group of 1—3 carbon atoms or a phenyl or benzyl group; $R_3$ is a hydrogen, fluroine or chlorine atom or an alkyl group of 1—3 carbon atoms, an alkoxyl group of 1—3 carbon atoms or an acyloxyl group of 1—3 carbon atoms; and $R_4$ is a hydrogen fluorine or chlorine atom or an alkyl group of 1—3 carbon atoms or an alkoxyl group which process comprises the catalytic hydrogenation of a compound of the formula (III):

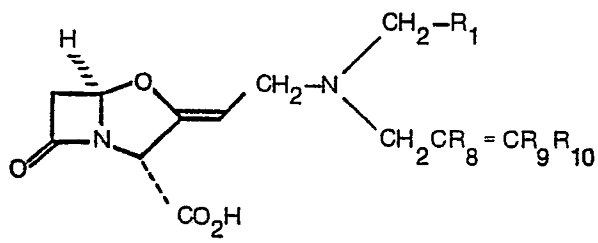

or a hydrogenolysable ester thereof wherein $R_1$ is as defined in relation to formula (I), or $R_1$ is $R_1$ where $R_1^1$ is a moiety that on hydrogenation provides a group $R_1$ as defined in relation to formula (I), $R_8$ is a hydrogen atom or a lower alkyl group, $R_9$ is a hydrogen atom or a lower alkyl group and $R_{10}$ is a hydrogen atom or a lower alkyl or phenyl group; wherein "lower alkyl" means an alkyl group of up to 4 carbon atoms.

2. A process as claimed in claim 1 for the preparation of a compound of the formula (I) wherein $R_1$ is a phenyl group.

3. A process as claimed in claim 1 for the preparation of a compound of the formula (I) wherein $R_1$ is a p-acetamidophenyl group.

4. A process as claimed in claim 1 for the preparation of a compound of the formula (I) wherein $CH_2R_1$ is an isobutyl group which process comprises the hydrogenation of a compound of the formula (III) wherein $CH_2R_1^1$ is an isobutenyl group.

5. A process as claimed in claim 1 for the preparation of a compound of the formula (I) wherein $CH_2R_1$ is an isobutyl group which process comprises the hydrogenation of a compound of the formula (III) wherein $CH_2R_1$ is an isobutyl group.

6. A process as claimed in any of the claims 1 to 5 wherein the moiety $CH_2CR_8{=}CR_9R_{10}$ is $CH_2C(CH_3){=}CH_2$, $CH_2C(C_2H_5){=}CH_2$, $CH_2C(nC_3H_7){=}CH_2$, $CH_2C(CH_3){=}CHCH_3$, $CH_2C(CH_3){=}C(CH_3)_2$, $CH_2C(CH_3){=}CHC_2H_5$ or $CH_2C(CH_3){=}CH.C_6H_5$.

7. A process according to any of claims 1 to 5 where $CH_2CR_8 = CR_9R_{10}$ is $CH_2C(CH_3){=}CH_2$ or $CH_2C(CH_3) = CHC_6H_5$.

8. A process as claimed in any of claims 1 to 7 wherein the esterifying radical is benzyl, p-bromobenzyl, p-chlorobenzyl, p-methylbenzyl or p-methoxybenzyl.

9. A process as claimed in any of claims 1 to 7 wherein the esterifying radical is 2-methylallyl, 2-ethylallyl, 2-n-propylallyl, 2-methyl-3-methylallyl, 2-methyl-3,3-dimethylallyl, 2-methyl-3-ethylallyl or 2-methyl-3-phenylallyl.

10. A process as claimed in claim 1 for the preparation of 9-*N*-isobutylaminodeoxyclavulanic acid which comprises the hydrogenation of benzyl 9-*N*-(isobutyl)-*N*-(2′-methylallyl)aminodeoxyclavulanate.

11. A process as claimed in claim 1 for the preparation of 9-*N*-isobutylaminodeoxyclavulanic acid which comprises the hydrogenation of benzyl 9-*N*,*N*-bis(2′-methylallyl)aminodeoxyclavulanate.

12. A process as claimed in any of claims 1 to 11 wherein the hydrogenation reaction is performed in the presence of a palladium metal catalyst.

13. A process as claimed in claim 12 wherein the palladium catalyst is palladium on charcoal, palladium on barium sulphate, palladium on calcium carbonate or palladium black.

14. A process as claimed in claim 13 wherein the catalyst is palladium on carbon with a proportion of palladium between 1% and 30%.

15. A process as claimed in claim 14 wherein the proportion of palladium is 10%.

16. A process as claimed in any of claims 1 to 15 wherein the pressure of hydrogen used is between $1.013 \times 10^5$ Pa (1 atmosphere) and $6.09 \times 10^5$ Pa (6 atmospheres).

17. A process as claimed in any of claims 1 to 16 wherein the pressure of hydrogen is $1.013 \times 10^5$ Pa (1 atmosphere).

18. A process as claimed in any of claims 1 to 17 wherein the reaction temperature is maintained between 0° and 30°C.

19. A process as claimed in any of claims 1 to 18 wherein the solvent for the hydrogenation reaction is ethanol, n-propanol, isopropanol, tetrahydrofuran, dioxan or ethyl acetate, or a mixture of these solvents in the presence of water.

20. A process as claimed in claim 19 wherein the solvent is ethanol.

21. A process as claimed in claim 19 wherein the solvent is isopropanol.

**Revendications pour les Etats Contractants BE CH DE FR GB IT LU NL SE**

1. Procédé pour la préparation d'un composé de formule (I):

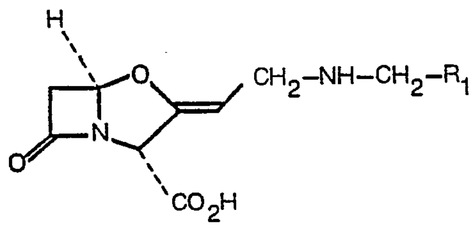

dans laquelle $R_1$ est un atome d'hydrogène, un groupe alcoyle ayant jusqu'à 5 atomes de carbone, un groupe cycloalcoyle ayant 5 ou 6 atomes de carbone, un groupe hydroxyalcoyle ayant jusqu'à 5 atomes de carbone ou une fraction molaire de sous-formule (a):

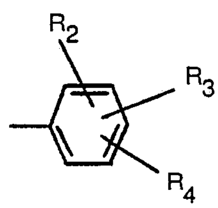

(a)

dans laquelle $R_2$ est un atome d'hydrogène, de fluor, de chlore ou de brome ou un groupe alcoyle ayant de 1 à 3 atomes de carbone, un groupe alcoxy ayant de 1 à 3 atomes de carbone, un groupe acyloxy ayant de 1 à 3 atomes de carbone, un groupe hydroxy, un groupe alcoxycarbonyle contenant de 1 à 3 atomes de carbone dans la fraction alcoxy ou un groupe $—N(R_5)CO.R_6$, $—N(R_5)SO_2R_6$ ou $—CO—NR_5R_6$, où $R_5$ est un atome d'hydrogène ou un groupe alcoyle ayant de 1 à 3 atomes de carbone, ou bien un groupe phényle ou benzyle, et $R_6$ est un groupe alcoyle ayant de 1 à 3 atomes de carbone ou un groupe phényle ou benzyle; $R_3$ est un atome d'hydrogène, de fluor ou de chlore ou un groupe alcoyle ayant de 1 à 3 atomes de carbone, un groupe alcoxy ayant de 1 à 3 atomes de carbone ou un groupe acyloxy ayant de 1 à 3 atomes de carbone; et $R_4$ est un atome d'hydrogène, de fluor ou de chlore ou un groupe alcoyle ayant de 1 à 3 atomes de carbone ou un groupe alcoxy, ce procédé consistant à effectuer l'hydrogénation catalytique d'un composé de formule (III):

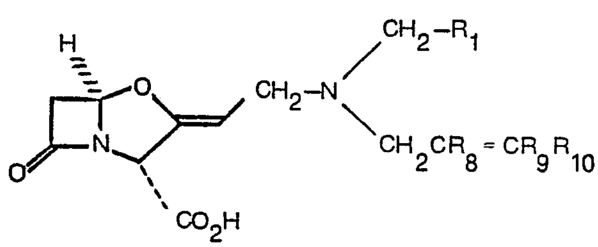

ou d'un ester hydrogénolysable de celui-ci, formule dans laquelle $R_1$ a la même signification que dans le cas de la formule (I) ou bien $R_1$ est $R_1^1$, où $R_1^1$ est une fraction molaire qui, par hydrogénation, fournit un groupe $R_1$ tel que défini dans le cas de la formule (I), $R_8$ est un atome d'hydrogène ou un groupe alcoyle inférieur, $R_9$ est un atome d'hydrogène ou un groupe alcoyle inférieur et $R_{10}$ est un atome d'hydrogène ou un groupe alcoyle inférieur ou phényle; où l'expression "alcoyle inférieur" désigne un groupe alcoyle ayant jusqu'à 4 atomes de carbone.

2. Procédé suivant la revendication 1 pour la préparation d'un composé de formule (I) dans laquelle $R_1$ est un groupe phényle.

3. Procédé suivant la revendication 1 pour la préparation d'un composé de formule (I) dans laquelle $R_1$ est un groupe p-acétamidophényle.

4. Procédé suivant la revendication 1 pour la préparation d'un composé de formule (I) dans laquelle $CH_2R_1$ est un groupe isobutyle, ce procédé consistant à effectuer l'hydrogénation d'un composé de formule (III) dans laquelle $CH_2R_1{}^1$ est un groupe isobutényle.

5. Procédé suivant la revendication 1 pour la préparation d'un composé de formule (I) dans laquelle $CH_2R_1$ est un groupe isobutyle, ce procédé consistant à effectuer l'hydrogénation d'un composé de formule (III) dans laquelle $CH_2R_1$ est un groupe isobutyle.

6. Procédé suivant l'une quelconque des revendications 1 à 5, caractérisé en ce que la fraction molaire $CH_2CR_8{=}CR_9R_{10}$ est $CH_2C(CH_3){=}CH_2$, $CH_2C(C_2H_5){=}CH_2$, $CH_2C(nC_3H_7){=}CH_2$, $CH_2C(CH_3){=}CHCH_3$, $CH_2C(CH_3){=}C(CH_3)_2$, $CH_2C(CH_3){=}CHC_2H_5$, ou $CH_2C(CH_3){=}CH.C_6H_5$.

7. Procédé suivant l'une quelconque des revendications 1 à 5, caractérisé en ce que

$CH_2R_8{=}CR_9R_{10}$ est $CH_2C(CH_3){=}CH_2$ ou
$CH_2C(CH_3){=}CHC_6H_5$.

8. Procédé suivant l'une quelconque des revendications 1 à 7, caractérisé en ce que le radical d'estérification est un groupe benzyle, p-bromobenzyle, p-chlorobenzyle, p-méthylbenzyle ou p-méthoxybenzyle.

9. Procédé suivant l'une quelconque des revendications 1 à 7, caractérisé en ce que le radical d'estérification est un groupe 2-méthylallyle, 2-éthylallyle, 2-n-propylallyle, 2-méthyl-3-méthylallyle, 2-méthyl-3,3-diméthylallyle, 2-méthyl-3-éthylallyle ou 2-méthyl-3-phénylallyle.

10. Procédé suivant la revendication 1 pour la préparation d'acide 9-N-isobutylaminodésoxy-clavulanique, caractérisé en ce qu'on effectue l'hydrogénation de 9-N-(isobutyl)-N-(2'-méthylallyl)-aminodésoxyclavulanate de benzyle.

11. Procédé suivant la revendication 1 pour la préparation d'acide 9-N-isobutylaminodésoxy-clavulanique, caractérisé en ce qu'on effectue l'hydrogénation de 9-N,N-bis(2'-méthylallyl)aminodésoxyclavulanate de benzyle.

12. Procédé suivant l'une quelconque des revendications 1 à 11, caractérisé en ce qu'on effectue la réaction d'hydrogénation en présence d'un catalyseur à base de palladium métal.

13. Procédé suivant la revendication 12, caractérisé en ce que le catalyseur au palladium est du palladium sur charbon, du palladium sur sulfate de baryum, du palladium sur carbonate de calcium ou du noir de palladium.

14. Procédé suivant la revendication 13, caractérisé en ce que le catalyseur est du palladium sur carbone renfermant une proportion de palladium comprise entre 1% et 30%.

15. Procédé suivant la revendication 14, caractérisé en ce que la proportion de palladium est égale à 10%.

16. Procédé suivant l'une quelconque des revendications 1 à 15, caractérisé en ce que la pression d'hydrogène utilisée est comprise entre $1{,}013 \times 10^5$ Pa (1 atmosphère) et $6{,}09 \times 10^5$ Pa (6 atmosphères).

17. Procédé suivant l'une quelconque des revendications 1 à 16, caractérisé en ce que la pression d'hydrogène est égale à $1{,}013 \times 10^5$ Pa (1 atmosphère).

18. Procédé suivant l'une quelconque des revendications 1 à 17, caractérisé en ce que la température de réaction est maintenue entre 0° et 30°C.

19. Procédé suivant l'une quelconque des revendications 1 à 18, caractérisé en ce que le solvant utilisé pour la réaction d'hydrogénation est de l'éthanol, du n-propanol, de l'isopropanol, du tétrahydrofuranne, du dioxanne ou de l'acétate d'éthyle, ou bien un mélange de ces solvants en présence d'eau.

20. Procédé suivant la revendication 19, caractérisé en ce que le solvant est de l'éthanol.

21. Procédé suivant la revendication 19, caractérisé en ce que le solvant est de l'isopropanol.

22. Composé de formule (III) ou ester hydrogénolysable de celui-ci, formule dans la quelle $R_1$, $R_9$ et $R_{10}$ sont tels que définis dans l'une quelconque des revendications 1 à 9 et $R_8$ est un groupe alcoyle ayant jusqu'à 4 atomes de carbone.

23. 9-N-(isobutyl-N-(2'-méthylallyl)aminodésoxyclavulanate de benzyle.

24. 9-N,N-bis-(2'-méthylallyl)aminodésoxyclavulanate de benzyle.

**Revendications pour l'Etat Contractant AT**

1. Procédé pour la préparation d'un composé de formule (I):

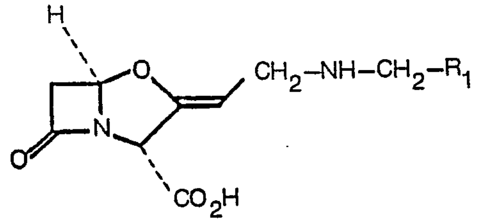

dans laquelle $R_1$ est un atome d'hydrogène, un groupe alcoyle ayant jusqu'à 5 atomes de carbone, un groupe cycloalcoyle ayant 5 ou 6 atomes de carbone, un groupe hydroxyalcoyle ayant jusqu'à 5 atomes de carbone ou une fraction molaire de sous-formule (a):

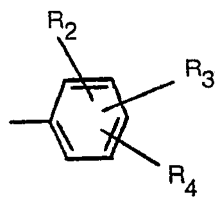

(a)

dans laquelle $R_2$ est un atome d'hydrogène, de fluor, de chlore ou de brome ou un groupe alcoyle ayant de 1 à 3 atomes de carbone, un groupe alcoxy ayant de 1 à 3 atomes de carbone, un groupe acyloxy ayant de 1 à 3 atomes de carbone, un groupe hydroxy, un groupe alcoxycarbonyle contenant de 1 à 3 atomes de carbone dans la fraction alcoxy ou un groupe $—N(R_5)CO.R_6$, $—N(R_5)SO_2R_6$ ou $—CO—NR_5R_6$, où $R_5$ est un atome d'hydrogène ou un groupe alcoyle ayant de 1 à 3 atomes de carbone, ou bien un groupe phényle ou benzyle, et $R_6$ est un groupe alcoyle ayant de 1 à 3 atomes de carbone ou un groupe phényle ou benzyle; $R_3$ est un atome d'hydrogène, de fluor ou de chlore ou un groupe alcoyle ayant de 1 à 3 atomes de carbone, un groupe alcoxy ayant de 1 à 3 atomes de carbone ou un groupe acyloxy ayant de 1 à 3 atomes de carbone; et $R_4$ est un atome d'hydrogène, de fluor ou de chlore ou un groupe alcoyle ayant de 1 à 3 atomes de carbone ou un groupe alcoxy, ce procédé consistant à effectuer l'hydrogénation catalytique d'un composé de formule (III):

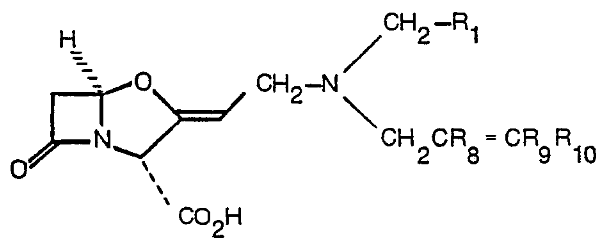

ou d'un ester hydrogénolysable de celui-ci, formule dans laquelle $R_1$ a la même signification que dans le cas de la formule (I) ou bien $R_1$ est $R_1{}^1$, où $R_1{}^1$ est une fraction molaire qui, par hydrogénation, fournit un groupe $R_1$ tel que défini dans le cas de la formule (I), $R_8$ est un atome d'hydrogène ou un groupe alcoyle inférieur, $R_9$ est un atome d'hydrogène ou un groupe alcoyle inférieur et $R_{10}$ est un atome d'hydrogène ou un groupe alcoyle inférieur ou phényle; où l'expression "alcoyle inférieur" désigne un groupe alcoyle ayant jusqu'à 4 atomes de carbone.

2. Procédé suivant la revendication 1 pour la préparation d'un composé de formule (I) dans laquelle $R_1$ est un groupe phényle.

3. Procédé suivant la revendication 1 pour la préparation d'un composé de formule (I) dans laquelle $R_1$ est un groupe p-acétamidophényle.

4. Procédé suivant la revendication 1 pour la préparation d'un composé de formule (I) dans laquelle $CH_2R_1$ est un groupe isobutyle, ce procédé consistant à effectuer l'hydrogénation d'un composé de formule (III) dans laquelle $CH_2R_1{}^1$ est un groupe isobutényle.

5. Procédé suivant la revendication 1 pour la préparation d'un composé de formule (I) dans laquelle $CH_2R_1$ est un groupe isobutyle, ce procédé consistant à effectuer l'hydrogénation d'un composé de formule (III) dans laquelle $CH_2R_1$ est un groupe isobutyle.

6. Procédé suivant l'une quelconque des revendications 1 à 5, caractérisé en ce que la fraction molaire $CH_2CR_8{=}CR_9R_{10}$ est $CH_2C(CH_3){=}CH_2$, $CH_2C(C_2H_5){=}CH_2$, $CH_2C(nC_3H_7){=}CH_2$, $CH_2C(CH_3){=}CHCH_3$, $CH_2C(CH_3){=}C(CH_3)_2$, $CH_2C(CH_3){=}CHC_2H_5$, ou $CH_2C(CH_3){=}CH.C_6H_5$.

7. Procédé suivant l'une quelconque des revendications 1 à 5, caractérisé en ce que

$CH_2R_8{=}CR_9R_{10}$ est $CH_2C(CH_3){=}CH_2$ ou $CH_2C(CH_3){=}CHC_6H_5$.

8. Procédé suivant l'une quelconque des revendications 1 à 7, caractérisé en ce que le radical d'estérification est un groupe benzyle, p-bromobenzyle, p-chlorobenzyle, p-méthylbenzyle ou p-méthoxybenzyle.

9. Procédé suivant l'une quelconque des revendications 1 à 7, caractérisé en ce que le radical d'estérification est un groupe 2-méthylallyle, 2-éthylallyle, 2-n-propylallyle, 2-méthyl-3-méthylallyle, 2-méthyl-3,3-diméthylallyle, 2-méthyl-3-éthylallyle ou 2-méthyl-3-phénylallyle.

10. Procédé suivant la revendication 1 pour la préparation d'acide 9-N-isobutylaminodésoxyclavulanique, caractérisé en ce qu'on effectue l'hydrogénation de 9-N-(isobutyl)-N-(2'-méthylallyl)-aminodésoxyclavulanate de benzyle.

11. Procédé suivant la revendication 1 pour la préparation d'acide 9-N-isobutylaminodésoxyclavulanique, caractérisé en ce qu'on effectue l'hydrogénation de 9-N,N-bis(2'-méthylallyl)aminodésoxyclavulanate de benzyle.

12. Procédé suivant l'une quelconque des revendications 1 à 11, caractérisé en ce qu'on effectue la réaction d'hydrogénation en présence d'un catalyseur à base de palladium métal.

13. Procédé suivant la revendication 12, caractérisé en ce que le catalyseur au palladium est du palladium sur charbon, du palladium sur sulfate de baryum, du palladium sur carbonate de calcium ou du noir de palladium.

14. Procédé suivant la revendication 13, caractérisé en ce que le catalyseur est du palladium sur carbone renfermant une proportion de palladium comprise entre 1% et 30%.

15. Procédé suivant la revendication 14, caractérisé en ce que la proportion de palladium est égale à 10%.

16. Procédé suivant l'une quelconque des revendications 1 à 15, caractérisé en ce que la pression d'hydrogène utilisée est comprise entre $1{,}013 \times 10^5$ Pa (1 atmosphère) et $6{,}09 \times 10^5$ Pa (6 atmosphères).

17. Procédé suivant l'une quelconque des revendications 1 à 16, caractérisé en ce que la pression d'hydrogène est égale à $1{,}013 \times 10^5$ Pa (1 atmosphère).

18. Procédé suivant l'une quelconque des revendications 1 à 17, caractérisé en ce que la température de réaction est maintenue entre 0° et 30°C.

19. Procédé suivant l'une quelconque des revendications 1 à 18, caractérisé en ce que le solvant utilisé pour la réaction d'hydrogénation est de l'éthanol, du n-propanol, de l'isopropanol, du tétrahydrofuranne, du dioxanne ou de l'acétate d'éthyle, ou bien un mélange de ces solvants en présence d'eau.

20. Procédé suivant la revendication 19, caractérisé en ce que le solvant est de l'éthanol.

21. Procédé suivant la revendication 19, caractérisé en ce que le solvant est de l'isopropanol.

**Patentansprüche für die Vertragsstaaten: BE CH DE FR GB IT LU NL SE**

1. Ein Verfahren zur Herstellung einer Verbindung der Formel (I)

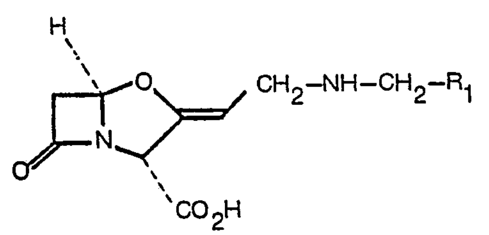

(I)

in welcher $R_1$ ein Wasserstoffatom, eine Alkylgruppe mit bis zu 5 Kohlenstoffatomen, eine Cycloalkylgruppe von 5 oder 6 Kohlenstoffatomen, eine Hydroxyalkylgruppe mit bis zu 5 Kohlenstoffatomen oder ein Molekülteil der Unterformel (a) ist:

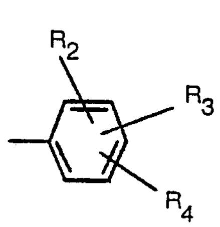

(a)

in welcher $R_2$ ein Wasserstoff-, Fluor-, Chlor- oder Bromatom oder eine Alkylgruppe mit 1 bis 3 Kohlenstoffatomen, eine Alkoxygruppe mit 1 bis 3 Kohlenstoffatomen, eine Acyloxygruppe mit 1 bis 3 Kohlenstoffatomen, eine Hydroxygruppe, eine Alkoxycarbonylgruppe enthaltend 1 bis 3 Kohlenstoffatome im Alkoxyteil oder eine Gruppe

$$—N(R_5)CO.R_6,\ —N(R_5)SO_2R_6 \text{ oder}$$

$—CO—NR_5R_6$ bedeutet, wobei $R_5$ ein Wasserstoffatom oder eine Alkylgruppe von 1 bis 3 Kohlenstoffatomen oder eine Phenyl- oder Benzylgruppe und $R_6$ eine Alkylgruppe mit 1 bis 3 Kohlenstoffatomen oder eine Phenyl- oder Benzylgruppe ist; $R_3$ ein Wasserstoffatom-, Fluor- oder Chloratom oder eine Alkylgruppe von 1 bis 3 Kohlenstoffatomen, eine Alkoxygruppe mit 1 bis 3 Kohlenstoffatomen oder eine Acyloxygruppe mit 1 bis 3 Kohlenstoffatomen bedeutet; und $R_4$ ein Wasserstoff-, Fluor- oder Chloratom oder eine Alkylgruppe von 1 bis 3 Kohlenstoffatomen oder eine Alkoxygruppe von 1 bis 3 Kohlenstoffatomen ist, welches Verfahren die katalytische Hydrierung einer Verbindung der Formel (III) oder eines hydrierbaren Esters derselben umfaßt.

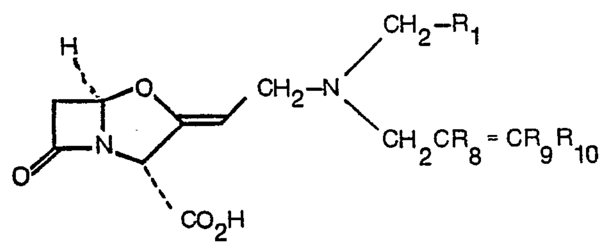

(III)

in welcher $R_1$ wie in Beziehung zu Formel (I) definiert ist oder $R_1$ $R_1^1$ ist, wobei $R_1^1$ einen Molekülteil bedeutet, der beim Hydrieren eine Gruppe $R_1$, wie in Beziehung zu Formel (I) definiert, liefert, $R_8$ ein Wasserstoffatom oder eine niedere Alkylgruppe, $R_9$ ein Wasserstoffatom oder eine niedere Alkylgruppe und $R_{10}$ ein Wasserstoffatom oder eine niedere Alkyl- oder Phenylgruppe ist, wobei "niederes Alkyl" eine Alkylgruppe mit bis zu 4 Kohlenstoffatomen bedeutet.

2. Ein Verfahren wie in Anspruch 1 beansprucht zur Herstellung einer Verbindung der Formel (I), in welcher $R_1$ eine Phenylgruppe ist.

3. Ein Verfahren wie in Anspruch 1 beansprucht zur Herstellung einer Verbindung der Formel (I), in welcher $R_1$ eine p-Acetamidophenylgruppe ist.

4. Ein Verfahren wie in Anspruch 1 beansprucht zur Herstellung einer Verbindung der Formel (I), in welcher $CH_2R_1$ eine Isobutylgruppe ist, welches Verfahren die Hydrierung einer Verbindung der Formel (III) umfaßt, in welcher $CH_2R_1^1$ eine Isobutenylgruppe ist.

5. Ein Verfahren wie in Anspruch 1 beansprucht zur Herstellung einer Verbindung der Formel (I), in welcher $CH_2R_1$ eine Isobutylgruppe ist, welches Verfahren die Hydrierung einer Verbindung der Formel (III) umfaßt, in welcher $CH_2R_1$ eine Isobutylgruppe ist.

6. Ein Verfahren wie in irgendeinem der Ansprüche 1 bis 5 beansprucht, in welchem der Molekülteil

$$CH_2CR_8{=}CR_9R_{10}$$

eine Gruppe $CH_2C(CH_3){=}CH_2$,

$$CH_2C(C_2H_5){=}CH_2,$$

$$CH_2C(nC_3H_7){=}CH_2,$$

$$CH_2C(CH_3){=}CHCH_3,$$

$$CH_2C(CH_3){=}C(CH_3)_2,$$

$$CH_2C(CH_3){=}CHC_2H_5 \text{ oder}$$

$CH_2C(CH_3){=}CH.C_6H_5$ bedeutet.

7. Ein Verfahren gemäß irgendeinem der Ansprüche 1 bis 5, in welchem $CH_2CR_8 = CR_9R_{10}$ die Gruppe $CH_2C(CH_3){=}CH_2$ oder $CH_2C(CH_3){=}CHC_6H_5$ bedeutet.

8. Ein Verfahren wie in irgendeinem der Ansprüche 1 bis 7 beansprucht, in welchem der veresternde Rest Benzyl, p-Brombenzyl, p-Chlorbenzyl, p-Methylbenzyl oder p-Methoxybenzyl ist.

9. Ein Verfahren wie in irgendeinem der Ansprüche 1 bis 7 beansprucht, in welchem der veresternde Rest 2-Methylallyl, 2-Äthylallyl, 2-n-Propylallyl, 2-Methyl-3-methylallyl, 2-Methyl-3,3-dimethylallyl, 2-Methyl-3-äthylallyl oder 2-Methyl-3-phenylallyl ist.

10. Ein Verfahren wie in Anspruch 1 beansprucht zur Herstellung von 9-*N*-Isobutylaminodeoxyclavulansäure, welches Verfahren die Hydrierung des 9-*N*-(Isobutyl)-*N*-(2′-methylallyl)aminodeoxyclavulansäurebenzylesters umfaßt.

11. Ein Verfahren wie in Anspruch 1 beansprucht zur Herstellung von 9-*N*-Isobutylaminodeoxyclavulansäure, welches die Hydrierung des 9-*N,N*-Bis(2′-methylallyl)aminodeoxyclavulansäurebenzylesters umfaßt.

12. Ein Verfahren wie in irgendeinem der Ansprüche 1 bis 11 beansprucht, in welchem die Hydrierungsreaktion in Anwesenheit eines Palladiummetallkatalysators durchgeführt wird.

13. Ein Verfahren wie in Anspruch 12 beansprucht, in welchem der Palladiumkatalysator Palladium auf Holzkohle, Palladium auf Bariumsulfat, Palladium auf Calciumcarbonat oder Palladiumschwarz ist.

14. Ein Verfahren wie in Anspruch 13 beansprucht, in welchem der Katalysator Palladium auf Kohlenstoff mit einem Anteil von Palladium zwischen 1% und 30% ist.

15. Ein Verfahren wie in Anspruch 14 beansprucht, in welchem der Anteil an Palladium 10% ist.

16. Ein Verfahren wie in irgendeinem der Ansprüche 1 bis 15 beansprucht, in welchem der verwendete Wasserstoffdruck zwischen $1{,}013 \times 10^5$ Pa (1 Atmosphäre) und $6{,}09 \times 10^5$ Pa (6 Atmosphären) liegt.

17. Ein Verfahren wie in irgendeinem der Ansprüche 1 bis 16 beansprucht, in welchem der Wasserstoffdruck $1{,}013 \times 10^5$ Pa (1 Atmosphäre) ist.

18. Ein Verfahren wie in irgendeinem der Ansprüche 1 bis 17 beansprucht, in welchem die Reaktionstemperatur zwischen 0 und 30°C gehalten wird.

19. Ein Verfahren wie in irgendeinem der Ansprüche 1 bis 18 beansprucht, in welchem das Lösungsmittel für die Hydrierungsreaktion Äthanol, n-Propanol, Isopropanol, Tetrahydrofuran, Dioxan oder Äthylacetat oder eine Mischung dieser Lösungsmittel in Anwesenheit von Wasser ist.

20. Ein Verfahren wie in Anspruch 19 beansprucht, in welchem das Lösungsmittel Äthanol ist.

21. Eine Verfahren wie in Anspruch 19 beansprucht, in welchem das Lösungsmittel Isopropanol ist.

22. Eine Verbindung der Formel (III) oder ein hydrierbarer Ester derselben, in welcher $R_1$, $R_9$ und $R_{10}$ wie in irgendeinem der Ansprüche 1 bis 9 definiert sind und $R_8$ eine Alkylgruppe mit bis zu 4 Kohlenstoffatomen ist.

23. 9-*N*-(Isobutyl)-*N*-(2′-methylallyl)aminodeoxyclavulansäurebenzylester.

24. 9-*N,N*-Bis(2′-méthylallyl)aminodeoxyclavulansäurebenzylester.

**Patentansprüche für die Vertragsstaat: AT**

1. Ein Verfahren zur Herstellung einer Verbindung der Formel (I)

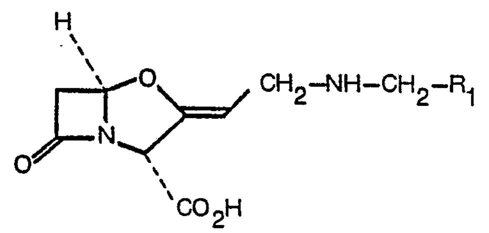

(I)

in welcher $R_1$ ein Wasserstoffatom, eine Alkylgruppe mit bis zu 5 Kohlenstoffatomen, eine Cycloalkylgruppe von 5 oder 6 Kohlenstoffatomen, eine Hydroxyalkylgruppe mit bis zu 5 Kohlenstoffatomen oder ein Molekülteil der Unterformel (a) ist:

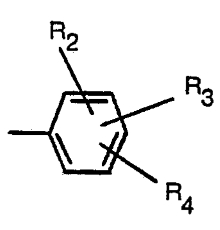

(a)

in welcher $R_2$ ein Wasserstoff-, Fluor-, Chlor- oder Bromatom oder eine Alkylgruppe mit 1 bis 3 Kohlenstoffatomen, eine Alkoxygruppe mit 1 bis 3 Kohlenstoffatomen, eine Acyloxygruppe mit 1 bis 3 Kohlenstoffatomen, eine Hydroxygruppe, eine Alkoxycarbonylgruppe enthaltend 1 bis 3 Kohlenstoffatome im Alkoxytyeil oder eine Gruppe

$$—N(R_5)CO.R_6, —N(R_5)SO_2R_6 \text{ oder}$$

—CO—$NR_5R_6$ bedeutet, wobei $R_5$ ein Wasserstoffatom oder eine Alkylgruppe von 1 bis 3 Kohlenstoffatomen oder eine Phenyl- oder Benzylgruppe und $R_6$ eine Alkylgruppe mit 1 bis 3 Kohlenstoffatomen oder eine Phenyl- oder Benzylgruppe ist; $R_3$ ein Wasserstoffatom-, Fluor- oder Chloratom oder eine Alkylgruppe von 1 bis 3 Kohlenstoffatomen, eine Alkoxygruppe mit 1 bis 3 Kohlenstoffatomen oder eine Acyloxygruppe mit 1 bis 3 Kohlenstoffatomen bedeutet; und $R_4$ ein Wasserstoff-, Fluor- oder Chloratom oder eine Alkylgruppe von 1 bis 3 Kohlenstoffatomen oder eine Alkoxygruppe von 1 bis 3 Kohlenstoffatomen ist, welches Verfahren die katalytische Hydrierung einer Verbindung der Formel (III) oder eines hydrierbaren Esters derselben umfaßt,

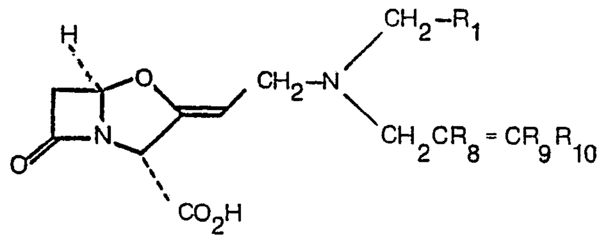

(III)

in welcher $R_1$ wie in Beziehung zu Formel (I) definiert ist oder $R_1$ $R_1^1$ ist, wobei $R_1^1$ einen Molekülteil bedeutet, der beim Hydrieren eine Gruppe $R_1$, wie in Beziehung zu Formel (I) definiert, liefert, $R_8$ ein Wasserstoffatom oder eine niedere Alkylgruppe, $R_9$ ein Wasserstoffatom oder eine niedere Alkylgruppe und $R_{10}$ ein Wasserstoffatom oder eine niedere Alkyl- oder Phenylgruppe ist, wobei "niederes Alkyl" eine Alkylgruppe mit bis zu 4 Kohlenstoffatomen bedeutet.

2. Ein Verfahren wie in Anspruch 1 beansprucht zur Herstellung einer Verbindung der Formel (I), in welcher $R_1$ eine Phenylgruppe ist.

3. Ein Verfahren wie in Anspruch 1 beansprucht zur Herstellung einer Verbindung der Formel (I), in welcher $R_1$ eine p-Acetamidophenylgruppe ist.

4. Ein Verfahren wie in Anspruch 1 beansprucht zur Herstellung einer Verbindung der Formel (I), in welcher $CH_2R_1$ eine Isobutylgruppe ist, welches Verfahren die Hydrierung einer Verbindung der Formel (III) umfaßt, in welcher $CH_2R_1^1$ eine Isobutenylgruppe ist.

5. Ein Verfahren wie in Anspruch 1 beansprucht zur Herstellung einer Verbindung der Formel (I), in welcher $CH_2R_1$ eine Isobutylgruppe ist, welches Verfahren die Hydrierung einer Verbindung der Formel (III) umfaßt, in welcher $CH_2R_1$ eine Isobutylgruppe ist.

6. Ein Verfahren wie in iregendeinem der Ansprüche 1 bis 5 beansprucht, in welchem der Molekülteil

$$CH_2CR_8{=}CR_9R_{10}$$

eine Gruppe $CH_2C(CH_3){=}CH_2$,

$CH_2C(C_2H_5){=}CH_2$,

$CH_2C(nC_3H_7){=}CH_2$,

$CH_2C(CH_3){=}CHCH_3$,

$CH_2C(CH_3){=}C(CH_3)_2$,

$CH_2C(CH_3){=}CHC_2H_5$ oder

$CH_2C(CH_3){=}CH.C_6H_5$ bedeutet.

7. Ein Verfahren gemäß irgendeinem der Ansprüche 1 bis 5, in welchem $CH_2CR_8 = CR_9R_{10}$ die Gruppe $CH_2C(CH_3){=}CH_2$ oder $CH_2C(CH_3){=}CHC_6H_5$ bedeutet.

8. Ein Verfahren wie in irgendeinem der Ansprüche 1 bis 7 beansprucht, in welchem der veresternde Rest Benzyl, p-Brombenzyl, p-Chlorbenzyl, p-Methylbenzyl oder p-Methoxybenzyl ist.

9. Ein Verfahren wie in irgendeinem der Ansprüche 1 bis 7 beansprucht, in welchem der veresternde Rest 2-Methylallyl, 2-Äthylallyl, 2-n-Propylallyl, 2-Methyl-3-methylallyl, 2-Methyl-3,3-dimethylallyl, 2-Methyl-3-äthylallyl oder 2-Methyl-3-phenylallyl ist.

10. Ein Verfahren wie in Anspruch 1 beansprucht zur Herstellung von 9-*N*-Isobutylaminodeoxyclavulansäure, welches Verfahren die Hydrierung des 9-*N*-(Isobutyl)-*N*-(2'-methylallyl)aminodeoxyclavulansäurebenzylesters umfaßt.

11. Ein Verfahren wie in Anspruch 1 beansprucht zur Herstellung von 9-*N*-Isobutylaminodeoxyclavulansäure, welches die Hydrierung des 9-*N*,*N*-Bis(2'-methylallyl)aminodeoxyclavulansäurebenzylesters umfaßt.

12. Ein Verfahren wie in irgendeinem der Ansprüche 1 bis 11 beansprucht, in welchem die Hydrierungsreaktion in Anwesenheit eines Palladiummetallkatalysators durchgeführt wird.

13. Ein Verfahren wie in Anspruch 12 beansprucht, in welchem der Palladiumkatalysator Palladium auf Holzkohle, Palladium auf Bariumsulfat, Palladium auf Calciumcarbonat oder Palladiumschwarz ist.

14. Ein Verfahren wie in Anspruch 13 beansprucht, in welchem der Katalysator Palladium auf Kohlenstoff mit einem Anteil von Palladium zwischen 1% und 30% ist.

15. Ein Verfahren wie in Anspruch 14 beansprucht, in welchem der Anteil an Palladium 10% ist.

16. Ein Verfahren wie in irgendeinem der Ansprüche 1 bis 15 beansprucht, in welchem der verwendete Wasserstoffdruck zwischen $1,013 \times 10^5$ Pa (1 Atmosphäre) und $6,09 \times 10^5$ Pa (6 Atmosphären) liegt.

17. Ein Verfahren wie in irgendeinem der Ansprüche 1 bis 16 beansprucht, in welchem der Wasserstoffdruck $1,013 \times 10^5$ Pa (1 Atmosphäre) ist.

18. Ein Verfahren wie in irgendeinem der Ansprüche 1 bis 17 beansprucht, in welchem die Reaktionstemperatur zwischen 0 und 30°C gehalten wird.

19. Ein Verfahren wie in irgendeinem der Ansprüche 1 bis 18 beansprucht, in welchem das Lösungsmittel für die Hydrierungsreaktion Äthanol, n-Propanol, Isopropanol, Tetrahydrofuran, Dioxan oder Äthylacetat oder eine Mischung dieser Lösungsmittel in Anwesenheit von Wasser ist.

20. Ein Verfahren wie in Anspruch 19 beansprucht, in welchem das Lösungsmittel Äthanol ist.

21. Eine Verfahren wie in Anspruch 19 beansprucht, in welchem das Lösungsmittel Isopropanol ist.